(19) Europäisches Patentamt
European Patent Office
Office européen des brevets

(11) Veröffentlichungsnummer: **0 200 947**
**B1**

(12) # EUROPÄISCHE PATENTSCHRIFT

(45) Veröffentlichungstag der Patentschrift: **12.09.90**

(51) Int. Cl.⁵: **C07D 233/56**, C07D 233/64, C07D 403/12, C07D 409/12, A61K 31/415

(21) Anmeldenummer: **86104950.0**

(22) Anmeldetag: **10.04.86**

(54) **1,3-Disubstituierte Imidazoliumsalze.**

(30) Priorität: **26.04.85 CH 1778/85**
**04.02.86 CH 410/86**

(43) Veröffentlichungstag der Anmeldung:
**17.12.86 Patentblatt 86/46**

(45) Bekanntmachung des Hinweises auf die Patenterteilung:
**12.09.90 Patentblatt 90/37**

(84) Benannte Vertragsstaaten:
**AT BE CH DE FR GB IT LI NL**

(56) Entgegenhaltungen:
**EP-A- 0 149 976**
**EP-B- 0 013 817**
**CH-A- 591 451**
**DD-A- 146 595**
**DE-A- 2 951 601**

**CHEMICAL ABSTRACTS, Band 97, Nr. 7, 16. August 1982, Columbus, Ohio, USA SUMITOMO CHEMICAL CO., LTD. "Alkylimidazole derivatives" Seite 651, Spalte 2, Zusammenfassung-Nr. 55 805z**
**R. Müssner, Diplomarbeit (1982), Universität von Innsbruck**

(73) Patentinhaber: **F. HOFFMANN-LA ROCHE AG, Postfach 3255, CH-4002 Basel(CH)**

(72) Erfinder: **Karpitschka, Eva Maria, Dr., Mariahilfpark 2, A-6020 Innsbruck(AT)**
Erfinder: **Klötzer, Wilhelm, Prof. Dr., Löfflerweg 5, A-6020 Innsbruck(AT)**
Erfinder: **Link, Helmut, Dr., Dammerkirchstrasse 70, CH-4056 Basel(CH)**
Erfinder: **Montavon, Marc, Dr., Realpstrasse 72, CH-4054 Basel(CH)**
Erfinder: **Müssner, Renate, Bachweg 11, FL-9485 Nendeln(LI)**

(74) Vertreter: **Notegen, Eric-André et al, Grenzacherstrasse 124 Postfach 3255, CH-4002 Basel(CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 200 947 B1

**Beschreibung**

Die vorliegende Erfindung betrifft Imidazoliumverbindungen. Die Verbindungen der allgemeinen Formel

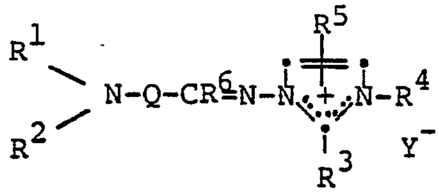

I

worin das Symbol Q eine 1,4-Phenylen- oder 1,4-Naphthylengruppe bedeutet, welche noch durch einen oder zwei Substituenten aus der Gruppe bestehend aus niederem Alkyl und niederem Alkoxy substituiert sein kann, $R^1$ und $R^2$ je Wasserstoff oder niederes Alkyl oder zusammen mit dem Stickstoffatom gegebenenfalls durch eine oder zwei niedere Alkylgruppen substituiertes 4-Morpholinyl, 1-Piperazinyl, 4-(niederes Alkyl)-1-piperazinyl, (4-niederes Alkoxycarbonyl)-1-piperazinyl oder 1-Pyrrolidinyl, $R^3$ niederes Alkyl, $R^4$ die Gruppe $-N=CR^6-Q-NR^1R^2$, $-N=CRc-Ra$ oder $-CH_2-CO-Rb$, $R^5$ Wasserstoff, niederes Alkyl, niederes Hydroxyalkyl, niederes Alkoxyalkyl, niederes Halogenalkyl, Phenyl oder einen ankondensierten Benzolring, $R^6$ Wasserstoff oder niederes Alkyl, Ra gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rb niederes Alkyl, niederes Alkoxy oder gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rc Wasserstoff oder niederes Alkyl, die gestrichelte Linie eine zusätzliche Doppelbindung und das Symbol $Y^-$ ein pharmazeutisch annehmbares Anion bedeuten, und die pharmazeutisch annehmbaren Säureadditionssalze davon besitzen wertvolle pharmakologische Eigenschaften. Sie besitzen insbesondere anthelmintische Eigenschaften.

Gegenstand der vorliegenden Erfindung sind: Die obigen Verbindungen der Formel I (sofern $R^3$ eine von Methyl verschiedene Bedeutung hat, wenn $R^4$ die Gruppe $-N=CH-Q-NR^1R^2$, $R^1$ und $R^2$ je Methyl, $R^5$ und $R^6$ je Wasserstoff, Q 1,4-Phenylen und Y Chlor bedeuten) und die pharmazeutisch annehmbaren Säureadditionssalze davon als solche; ein Verfahren und Zwischenprodukte zu deren Herstellung; die Verwendung der Zwischenprodukte zur Herstellung von therapeutischen Wirkstoffen; die obigen Verbindungen der Formel I und die pharmazeutisch annehmbaren Säureadditionssalze davon zur Anwendung als therapeutische Wirkstoffe; Arzneimittel auf der Basis von Verbindungen der Formel I und deren pharmazeutisch annehmbaren Säureadditionssalzen und deren Herstellung; die Verwendung dieser Verbindungen bei der Bekämpfung oder Verhütung von Krankheiten; sowie die Verwendung dieser Verbindung zur Herstellung von anthelmintisch wirksamen Mitteln.

In einer Diplomarbeit aus dem Jahre 1982 von R. Müssner, Universität Innsbruck, wird 1,3-Bis[[p-(dimethylamino]benzyliden]amino]-2-methylimidazoliumchlorid, eine Verbindung der obigen Formel I, und dessen Herstellung aus 1,3-Diamino-2-methylimidazoliumchlorid, eine Verbindung der weiter unten folgenden Formel II, beschrieben.

In der EP-A 149 976 werden 2,$\alpha$-di(nieder Alkyl)-1-benzylimidazole zur Behandlung von depressiven Zuständen beschrieben.

Der Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie "Alkylgruppe", "Alkoxy" und "Alkylthio", bezeichnet geradkettige oder verzweigte, gesättigte Kohlenwasserstoffreste, wie Methyl, Aethyl, n-Propyl, i-Propyl, n-Butyl, s-Butyl, i-Butyl und t-Butyl.

Der Ausdruck "Halogen" bezeichnet die vier Formen Fluor, Chlor, Brom und Jod.

In einer speziellen Ausführungsform betrifft die vorliegende Erfindung Verbindungen der obigen Formel I, worin $R^4$ die Gruppe $-N=CR^6-Q-NR^1R^2$ bedeutet, d.h. Verbindungen der allgemeinen Formel

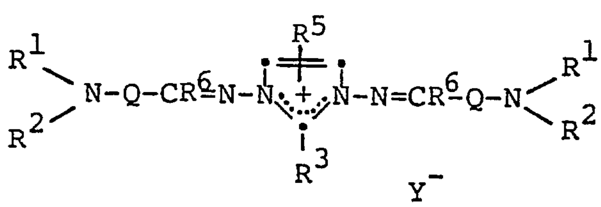

Ia

worin $R^1$, $R^2$, $R^3$, $R^5$, $R^6$, Q und $Y^-$ obige Bedeutung besitzen, wobei die Symbole Q, $R^1$, $R^2$ und $R^6$ jeweils gleich oder verschieden sein können.

In einer besonders bevorzugten Ausführungsform bedeutet das Symbol Q 1,4-Phenylen.

In einer besonders bevorzugten Ausführungsform bedeuten $R^1$ und $R^2$ je niederes Alkyl.

$R^3$ bedeutet vorzugsweise niederes Alkyl mit mindestens 2 Kohlenstoffatomen.

Sofern das Symbol $R^4$ nicht die Gruppe $-N{=}CR^6{-}Q{-}NR^1R^2$ bedeutet, bedeutet es vorzugsweise die Gruppe –N=CRc–Ra oder $-CH_2-CO-Rb$.

Die Symbole $R^5$, $R^6$ und Rc bedeuten vorzugsweise Wasserstoff.

Die nachfolgend aufgeführten Imidazoliumsalze sind Vertreter der durch die allgemeine Formel I definierten Stoffklasse:

3-[p-(Chlorbenzyliden)amino]-1-[[p-(dimethylamino)benzyliden]amino]-2-äthylimidazoliumsalze,
3-[p-(Dimethylamino)phenacyl]-1-[[p-(dimethylamino)benzyliden]amino]-2-äthylimidazoliumsalze,
1,3-Bis[[(p-(dimethylamino)benzyliden]amino]-2-methylimidazoliumsalze,
1,3-Bis[[(p-(dimethylamino)benzyliden]amino]-2-äthylimidazoliumsalze,
1-[[p-(Dimethylamino)benzyliden]amino]-2-äthyl-3-[(p-nitrobenzyliden)amino]imidazoliumsalze und
1,3-Bis[[(p-(dimethylamino)benzyliden]amino]-2-propylimidazoliumsalze.

Die neuen Verbindungen der Formel I und ihre pharmazeutisch annehmbaren Säureadditionssalze können erfindungsgemäß hergestellt werden, indem man

a) eine Verbindung der allgemeinen Formel

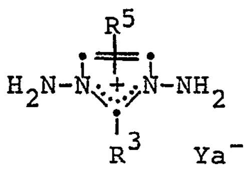

II

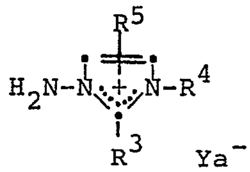

III

oder

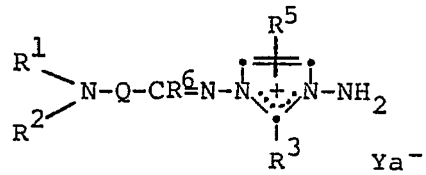

IIIa'

worin $R^1$, $R^2$, $R^3$, $R^4$, $R^5$, $R^6$ und Q obige Bedeutung besitzen, und $Ya^-$ ein Anion bedeutet, mit einer Carbonylverbindung der allgemeinen Formel

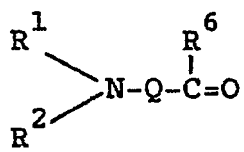

IVa

worin $R^1$, $R^2$, $R^6$ und Q obige Bedeutung besitzen, oder eine Verbindung der obigen Formel IIIa' mit einer Carbonylverbindung der allgemeinen Formel

Ra–C(Rc)=O IVb

worin Ra und Rc obige Bedeutung besitzen, umsetzt, oder

b) eine Verbindung der allgemeinen Formel

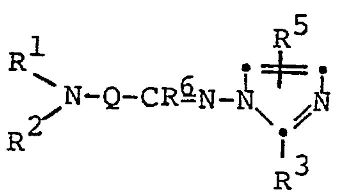

Va'

worin $R^1$, $R^2$, $R^3$, $R^5$, $R^6$ und Q obige Bedeutung besitzen, mit einer Verbindung der allgemeinen Formel

$R^{41}$–X VI

worin $R^{41}$ die Gruppe $-CH_2-CO-Rb$ und X eine Abgangsgruppe bedeuten, und Rb obige Bedeutung besitzt, umsetzt,

c) gegebenenfalls in einer erhaltenen Verbindung das mit $Ya^-$ bezeichnete Anion gegen ein pharmazeutisch annehmbares Anion austauscht und

d) erwünschtenfalls eine erhaltene Verbindung der Formel I in ein pharmazeutisch annehmbares Salz überführt.

Bei verschiedenen der obigen erfindungsgemäßen Verfahren ist es notwendig, die in den Ausgangsstoffen allfällig vorhandenen reaktionsfähigen Amino- und/oder Hydroxylgruppen durch Schutzgruppen zu blockieren. Diese Fälle sind für den Fachmann ohne weiteres erkennbar, und auch die Auswahl der jeweils geeigneten Schutzgruppen bereitet ihm keine Schwierigkeiten.

Bei der Umsetzung von Aminen mit Aldehyden oder Ketonen gemäß Verfahrensvariante a) handelt es sich um eine an sich bekannte und jedem Fachmann geläufige Reaktion. Als Lösungsmittel eignen sich beispielsweise niedere Fettsäuren, wie Essigsäure und Propionsäure, niedere Alkohole, wie Methanol, Äthanol und 2-Propanol, niedere Fettsäureester, wie Essigester, niedere Äther, wie Diäthyläther, t-Butylmethyläther, Äthylenglykoldimethyläther, Tetrahydrofuran und Dioxan, halogenierte niedere Kohlenwasserstoffe, wie Methylenchlorid, Chloroform und Äthylenchlorid, aromatische Kohlenwasserstoffe, wie Benzol, Toluol und Xylol, Acetonitril, N,N-Dimethylformamid und Dimethylsulfoxid. Die Reaktionstemperatur ist nicht kritisch. Die Reaktion kann beispielsweise in einem Bereich von etwa 0°C bis zur Siedetemperatur des gewählten Lösungsmittels durchgeführt werden. Man arbeitet jedoch vorzugsweise bei Raumtemperatur. Bei der Umsetzung mit den weniger reaktiven Ketonen der Formel IV (Rc= niederes Alkyl) verwendet man vorzugsweise ein Kondensationsmittel, wie Triäthyloxoniumtetrafluoroborat.

Gemäß Verfahrensvariante b) können Verbindungen der Formel I, worin $R^4$ die Gruppe $-CH_2-CO-Rb$ bedeutet durch Alkylierung von Imidazolderivaten der Formel Va′ hergestellt werden. Es handelt sich auch hierbei um eine an sich bekannte und jedem Fachmann geläufige Reaktion. Geeignete Lösungsmittel sind beispielsweise halogenierte niedere Kohlenwasserstoffe, wie Methylenchlorid, Chloroform und Äthylenchlorid, offenkettige oder cyclische Äther, wie Diäthyläther, t-Butylmethyläther, Äthylenglykoldimethyläther, Tetrahydrofuran und Dioxan, niedere Fettsäureester, wie Essigester, niedere Alkohole, wie Methanol, Äthanol und i-Propanol, aromatische Kohlenwasserstoffe, wie Benzol, Toluol und Xylol, Acetonitril, N,N-Dimethylformamid und Dimethylsulfoxid. Die Reaktionstemperatur ist nicht kritisch. Man kann beispielsweise in einem Bereich von etwa 0°C bis zur Siedetemperatur des Lösungsmittels arbeiten.

Gemäß Verfahrensvariante c) kann man in einer erhaltenen Verbindung das mit $Ya^-$ bezeichnete Anion gegen ein pharmazeutisch annehmbares Anion austauschen. Auch bei dieser Austauschreaktion handelt es sich um eine an sich bekannte und jedem Fachmann geläufige Reaktion. Man verwendet vorzugsweise herkömmliche Ionenaustauscher, welche mit einem pharmazeutisch annehmbaren Anion beladen sind.

Gemäß Verfahrensvariante d) können Verbindungen der Formel I in pharmazeutisch annehmbare Säureadditonssalze übergeführt werden. Derartige Säureadditionssalze können nach an sich bekannten und jedem Fachmann geläufigen Methoden hergestellt werden. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succininate, Methansulfonate, p-Toluolsulfonate und dergleichen.

Die verschiedenen als Ausgangsstoffe erwähnten Verbindungen können gemäß dem nachfolgenden Reaktionsschema I hergestellt werden, worin $R^3$, $R^{41}$, $R^5$, Ra, Rc, R und $Ya^-$ obige Bedeutung besitzen.

## Reaktionsschema I

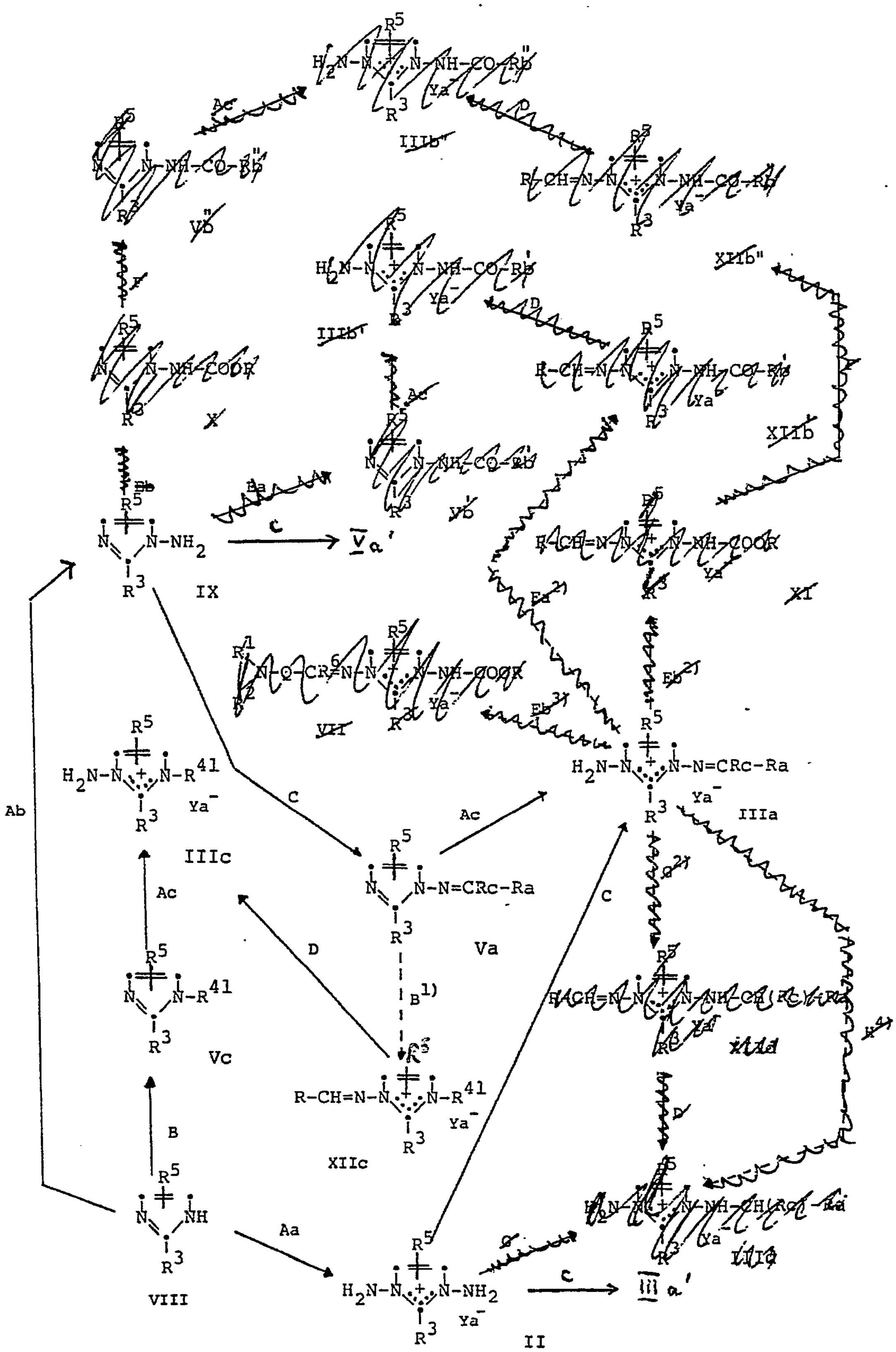

Die gestrichelten Pfeile bedeuten, daß nicht alle der angegebenen Ausgangsstoffe für die fragliche Reaktion in Frage kommen; vgl. die nachfolgenden Angaben zur Bezugsziffer 1).

1) Ausgangsstoffe für diese Reaktion sind Verbindungen der Formel Va, worin Ra gegebenenfalls durch niederes Alkyl, niederes Alkoxy oder Halogen substituiertes Phenyl und Rc Wasserstoff bedeuten.

Bei den nachstehend näher erläuterten Reaktionsschritten A bis D handelt es sich durchwegs um an sich bekannte und jedem Fachmann geläufige Reaktionen.

Aa: Bei diesem Reaktionsschritt handelt es sich um eine elektrophile Aminierung mit einem elektrophilen Aminierungsmittel wie Hydroxylamin-O-sulfonsäure oder dessen Salzen mit anorganischen Basen. Vorzugsweise verwendet man die entsprechenden Alkalimetallsalze, beispielsweise das Natriumsalz, und arbeitet in wässeriger Lösung. Man erhält dabei in der Regel ein Gemisch bestehend aus dem Diamin der Formel II und dem Monoamin der Formel IX. Die beiden Verbindungen können nach an sich bekannten und jedem Fachmann geläufigen Methoden voneinander getrennt werden. Die weiter unten folgenden Beispiele enthalten detaillierte Angaben betreffend die Auftrennung der erhaltenen Gemische.

Ab: Bei diesem Reaktionsschritt handelt es sich um eine elektrophile Aminierung mit einem elektrophilen Aminierungsmittel wie O-Diphenylphosphinylhydroxylamin, wobei man die Verbindung der Formel VIII vor der Umsetzung mit dem elektrophilen Aminierungsmittel mit einer starken Base in ein Alkalimetallsalz überführt. Geeignete Basen sind beispielsweise niedere Alkalimetallalkoxide, wie Natriummethoxid und Natriumäthoxid, und Alkalimetallhydride, wie Natriumhydrid. Geeignete Lösungsmittel sind beispielsweise N-Methylpyrrolidon, N,N-Dimethylformamid, niedere Alkohole, Äther, wie Tetrahydrofuran, Diäthylenglykoldimethyläther, Diäthylenglykoldiäthyläther und Diäthylenglykoldibutyläther. Die Reaktionstemperatur liegt vorzugsweise in einem Bereich von etwa 0°C bis 100°C, wobei man vorzugsweise bei Raumtemperatur arbeitet.

Ac: Bei diesem Reaktionsschritt handelt es sich um eine elektrophile Aminierung mit einem elektrophilen Aminierungsmittel wie O-Diphenylphosphinylhydroxylamin. Geeignete Lösungsmittel sind beispielsweise halogenierte niedere Kohlenwasserstoffe, wie Chloroform, Methylenchlorid und Äthylenchlorid, Äther, wie Diäthyläther und Tetrahydrofuran, niedere Alkohole, wie Äthanol, Acetonitril, N,N-Dimethylformamid, Dimethylsulfoxid und Mischungen davon. Die Reaktionstemperatur liegt in einem Bereich von etwa 0°C bis zur Siedetemperatur des gewählten Lösungsmittels. Man arbeitet vorzugsweise bei Raumtemperatur.

B: Bei diesem Reaktionsschritt wird das entsprechende Ausgangsmaterial mit einer Verbindung der obigen Formel VI umgesetzt. Geeignete Lösungsmittel sind beispielsweise halogenierte niedere Kohlenwasserstoffe, wie Methylenchlorid, Chloroform und Äthylenchlorid, offenkettige und cyclische Äther, wie Diäthyläther, Diisopropyläther, Äthylenglykoldimethyläther, Diäthylenglykoldiäthyläther, Tetrahydrofuran und Dioxan, Acetonitril und N,N-Dimethylformamid. Diese Reaktion kann in einem Temperaturbereich von etwa 0°C bis zur Siedetemperatur des gewählten Lösungsmittels durchgeführt werden.

C: Bei diesem Reaktionsschritt wird das entsprechende Ausgangsmaterial mit einem Aldehyd oder Keton der obigen Formel IV zum entsprechenden Aldimin bzw. Ketimin umgesetzt. Geeignete Lösungsmittel sind beispielsweise niedere Fettsäuren, wie Essigsäure und Propionsäure, niedere Alkohole, wie Methanol, Äthanol und 2-Propanol, halogenierte niedere Kohlenwasserstoffe, wie Methylenchlorid, Chloroform und Äthylenchlorid, offenkettige und cyclische Äther wie Diäthyläther, Diisopropyläther, t-Butylmethyläther, Äthylenglykoldimethyläther, Diäthylenglykoldiäthyläther, Tetrahydrofuran und Dioxan, Acetonitril, N,N-Dimethylformamid und Dimethylsulfoxid. Die Reaktion kann in einem Temperaturbereich von etwa 0°C bis zur Siedetemperatur des gewählten Lösungsmittels durchgeführt werden. Man arbeitet vorzugsweise bei Raumtemperatur. Bei der Umsetzung mit den weniger aktiven Ketonen der Formel IV (Rc= niederes Alkyl) verwendet man vorzugsweise ein Kondensationsmittel, wie Triäthyloxoniumtetrafluoroborat.

D: Bei diesem Reaktionsschritt handelt es sich um die Hydrolyse eines Aldimins. In einer bevorzugten Ausführungsform behandelt man das entsprechende Ausgangsmaterial mit einer wäßrigen Säure und entfernt den erhaltenen aromatischen Aldehyd mittels Wasserdampfdestillation. Geeignete Säuren sind beispielsweise verdünnte Salzsäure und verdünnte Bromwasserstoffsäure.

Die neuen, als Ausgangsstoffe verwendeten und oben beschriebenen Verbindungen und deren Verwendung zur Herstellung von Verbindungen der allgemeinen Formel I sind ebenfalls Gegenstand der vorliegenden Erfindung. Es handelt sich dabei um die oben definierten Verbindungen der Formel II und III (sofern $R^3$ eine von Methyl verschiedene Bedeutung hat) und um die Verbindungen der allgemeinen Formel

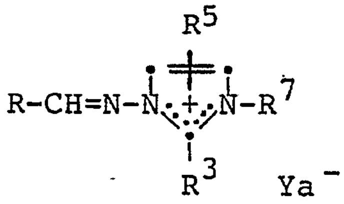

und

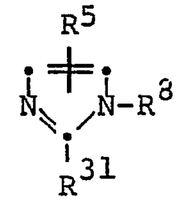

XV XVI'

worin $R^7$ die Gruppe $-CH_2-CO-Rb$, $R^8$ die Gruppe $-N{=}CR^6-Q-NR^1R^2$ oder $-N{=}CRc-Ra$ und $R^{31}$ niederes Alkyl mit mindestens zwei Kohlenstoffatomen bedeuten, und R, $R^1$, $R^2$, $R^3$, $R^5$, $R^6$, Ra, Rb, Rc, $Ya^-$ und Q obige Bedeutung besitzen.

Wie bereits eingangs erwähnt besitzen die Verbindungen der Formel I wertvolle pharmakologische Eigenschaften. Sie weisen beispielsweise antiparasitäre Eigenschaften auf und sind insbesondere wirksam gegen parasitäre Würmer. Besonders ausgeprägt ist ihre Wirkung gegen parasitäre Würmer, insbesondere gegen Nematoden wie die Filarien. Diese pharmakologischen Eigenschaften können mittels bekannter und jedem Fachmann geläufiger Testmethoden ermittelt werden.

Die filarizide Wirkung der Verbindungen der Formel I kann beispielsweise an Baumwollratten (Sigmodon hispidus) ermittelt werden, welche mit Litomosoides carinii infiziert worden sind. Die Infektion mit L. carinii wird durch die blutsaugende Milbe Bdellonyssus bacoti übertragen, in welcher sich die Entwicklung von der Mikrofilarie zur infektiösen Larve vollzieht. Die Baumwollratten werden infiziert, indem man sie dem Biss infizierter Milben aussetzt. 14 Wochen nach der Infektion werden Gruppen von 2–4 Tieren subkutan mit der zu testenden Verbindung behandelt. 42 Tage nach Behandlung mit der zu testenden Verbindung werden die Versuchstiere seziert, wobei man die addulten Filarien aus der Pleuralhöhle entfernt. Lebende und tote oder eingekapselte Würmer werden voneinander getrennt und gewogen. Die filarizide Wirkung wird ausgedrückt als prozentualer Anteil toter Makrofilarien pro Behandlungsgruppe. Mit den Werten aus verschiedenen Dosierungsgruppen wird dann die $ED_{90}$ mittels Probitanalyse bestimmt. Die $ED_{90}$ ist diejenige Dosis, bei welcher 90% der aus der Pleuralhöhle entfernten Würmer tot sind. In der nachfolgenden Tabelle werden die Resultate zusammengestellt, welche mit repräsentativen Vertretern der durch die allgemeine Formel I definierten Verbindungsklasse in dem vorstehend geschilderten Versuch erhalten worden sind. Die Tabelle enthält außerdem Angaben über die akute Toxizität einiger dieser Verbindungen in mg pro kg bei einmaliger oraler Verabreichung an Mäusen.

Tabelle

| Verbindung der Formel I, worin $R^5$ und $R^6$ = Wasserstoff und worin: $R^1$ | $R^2$ | Q | $R^3$ | $R^4$ | $Y^-$ | $ED_{90}$ (mg/kg s.c.) | $DL_{50}$ (mg/kg p.o.) |
|---|---|---|---|---|---|---|---|
| $CH_3$- | $CH_3$- | 1,4-Phenylen | $CH_3$- | 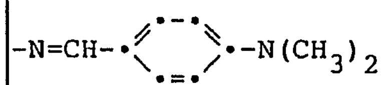 | $Cl^-$ | 1,6 | 80-156 |
| " | " | " | " | " | $CH_3COO^-$ | 0,85 | - |
| " | " | " | $C_2H_5$- | " | $Cl^-$ | 0,4 | 62,5-125 |
| " | " | " | " | " | $CH_3COO^-$ | 0,25 | - |
| " | " | " | $CH_3(CH_2)_2$- | " | $Cl^-$ | <0,25 | 62,5-125 |
| " | " | " | $CH_3(CH_2)_3$- | " | " | <0,25 | - |
| " | " | " | $C_2H_5$- | 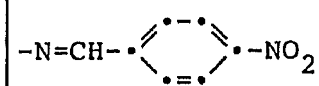 | " | <1 | – |
| $C_2H_5$- | $C_2H_5$- | " | " | 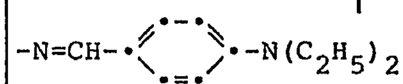 | " | 0,75 | - |
| $CH_3$- | $CH_3$- | 1,4-Naphthylen | " | 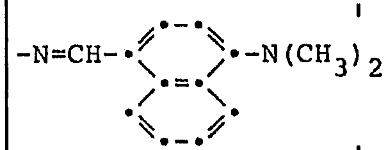 | " | 3 | 312-625 |

Die Verbindungen der Formel I können als Heilmittel, z.B. in Form pharmazeutischer Präparate zur enteralen oder parenteralen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, daß man die beschriebenen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Öle, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Öle. Für Suppositorien eignen sich als Trägermaterialien beispielsweise natürliche oder gehärtete Öle, Wachse, Fette und halbflüssige oder flüssige Polyole.

Als pharmazeutische Hilfsstoffe kommen die üblichen Stabilisierungs-, Konservierungs-, Netz- und Emulgiermittel, Mittel zur geschmacklichen Verbesserung, Salze zur Veränderung des osmotischen Druckes, Puffersubstanzen, Lösungsvermittler, Färbe- und Überzugsmittel und Antioxidantien in Frage.

Die Dosierung der beschriebenen Stoffe kann, abhängig von der zu behandelnden Krankheit, dem Alter und dem individuellen Zustand des Patienten und von der Applikationsweise, innerhalb weiter Grenzen variieren und ist natürlich in jedem einzelnen Fall den individuellen Gegebenheiten anzupassen. Für die Prophylaxe und Therapie von Infektionskrankheiten, welche durch Bakterien, Pilze oder Parasiten hergerufen werden, kommt für den erwachsenen Patienten eine tägliche Dosis von etwa 0,01 g bis etwa 4 g, insbesondere etwa 0,05 g bis etwa 2 g in Betracht. Je nach Dosierung ist es dabei zweckmäßig, die Tagesdosis in mehreren Dosierungseinheiten zu verabreichen.

Die erfindungsgemäßen pharmazeutischen Präparate enthalten zweckmäßigerweise etwa 10–1000 mg, vorzugsweise 50–500 mg eines erfindungsgemäßen Stoffes.

Die nachfolgenden Beispiele dienen der näheren Erläuterung der vorliegenden Erfindung. Sämtliche Temperaturen sind in Celsiusgraden angegeben.

Beispiel 1

a) Eine Lösung von 21,2 g (211 mMol) 2-Äthylimidazol in 70 ml Wasser wird innert 30 Minuten mit einer bei 0°C hergestellten Lösung von 25,0 g (221 mMol) Hydroxylamin-O-sulfonsäure und 18,5 g (220 mMol) Natriumhydrogencarbonat in 150 ml Wasser versetzt. Die Reaktionslösung erwärmt sich dabei auf ca. 30°C. Man rührt während 20 Stunden bei Raumtemperatur, säuert mit 65 ml 2N-Salzsäure an, gibt eine Lösung von 15,5 g (146 mMol) Benzaldehyd in 50 ml Äther dazu und rührt während 6 Stunden. Das ausgefallene Produkt wird abfiltriert und aus Methanol umkristallisiert. Man erhält 1,3-Bis(benzylidenamino)-2-äthyl-imidazoliumbenzaldoxim-O-sulfonat vom Schmelzpunkt 180–182°.

Die Mutterlauge wird mit Äther gewaschen, mit 3N-Natronlauge neutralisiert und mit Methylenchlorid extrahiert. Nach Trocknen des Auszugs über Natriumsulfat, Eindampfen und Kristallisieren des so erhaltenen Materials aus Äther/Petroläther erhält man 1-Benzylidenamino-2-äthylimidazol vom Schmelzpunkt 67–68°.

2,5 g (7,4 mMol) 1,3-Bis(benzylidenamino)-2-äthyl-imidazolium-benzaldoxim-O-sulfonat, 20 ml Wasser und 9,2 ml 2N-Salzsäure werden auf dem Dampfbad erwärmt, wobei man den entstehenden Benzaldehyd mittels Wasserdampfdestillation entfernt. Die erhaltene Lösung wird im Vakuum eingedampft. Der verbleibende Rückstand wird auf eine mit 20 g Amberlite™ IRA 400 (Chlorid) beladene Säule gebracht, worauf man mit Wasser eluiert. Die wäßrige Lösung wird eingedampft (zuletzt im Hochvakuum), und der Rückstand wird aus Äthanol/Äther kristallisiert. Man erhält 1,3-Diamino-2-äthyl-imidazoliumchlorid als weiße Kristalle vom Schmelzpunkt 181–183°.

Eine Lösung von 19,9 g (0,1 Mol) 1-Benzylidenamino-2-äthyl-imidazol in 200 ml 1,5N-Salzsäure wird solange einer Wasserdampfdestillation unterworfen, bis kein Benzaldehyd mehr entsteht. Es wird eingedampft, und das erhaltene Produkt wird aus Äthanol/Äther umkristallisiert. Man erhält 14,5 g 1-Amino-2-äthylimidazol-Hydrochlorid vom Schmelzpunkt 90–91°.

In analoger Weise erhält man:

b) 705 mg (4,7 mMol) 4-Dimethylaminobenzaldehyd werden zu einer Lösung von 348 mg (2,1 mMol) 1,3-Diamino-2-äthyl-imidazoliumchlorid in 4,4 ml Eisessig gegeben, worauf man während 20 Stunden rührt.

Die erhaltenen gelben Kristalle werden abfiltriert und aus Äthanol umkristallisiert. Man erhält 1,3-Bis[[p-(dimethylamino)benzyliden]amino]-2-äthyl-imidazoliumchlorid vom Schmelzpunkt 248°.

In analoger Weise erhält man:

c) Aus 1,3-Diamino-2-äthylimidazoliumchlorid und 4-Diäthylaminobenzaldehyd das 1,3-Bis[[p-(diäthylamino)benzyliden]-amino]-2-äthyl-imidazoliumchlorid vom Schmelzpunkt 225–226° (aus Äthanol/Äther);

d) aus 1,3-Diamino-2-äthylimidazoliumchlorid und 4-Dimethylamino-3,5-dimethoxy-benzaldehyd das 1,3-Bis[[4-(dimethylamino)-3,5-dimethoxybenzyliden]amino]-2-äthyl-imidazoliumchlorid vom Schmelzpunkt 223–224° (aus Äthanol/Äther);

e) aus 1,3-Diamino-2-äthylimidazoliumchlorid und 4-Amino-3,5-dimethoxy-benzaldehyd das 1,3-Bis[(4-amino-3,5-dimethoxybenzyliden)amino]-2-äthyl-imidazoliumchlorid vom Schmelzpunkt 226–228° (aus Äthanol/Äther);

f) aus 1,3-Diamino-2-äthylimidazoliumchlorid und p-(4-Methyl-1-piperazinyl)benzaldehyd das 2-Äthyl-1,3-bis[[p-(4-methyl-1-piperazinyl)benzyliden]amino]-imidazoliumchlorid vom Schmelzpunkt 242° (aus Äthanol/Äther);

g) aus 1,3-Diamino-2-äthylimidazoliumchlorid und p-(N-Morpholino)benzaldehyd das 2-Äthyl-1,3-bis[(p-(N-morpholinobenzyliden)amino]imidazoliumchlorid vom Schmelzpunkt 244–245° (aus Äthanol);

h) aus 1,3-Diamino-2-äthylimidazoliumchlorid und 4-Dimethylamino-1-naphthalin-carboxaldehyd das 1,3-Bis[[[4-(dimethylamino)-1-naphthyl]methyliden]amino]-2-äthyl-imidazoliumchlorid vom Schmelzpunkt 215° (aus Äthanol/Äther);

i) aus 1,3-Diamino-2-äthylimidazoliumchlorid und 2-(Dimethylamino)-5-methylbenzaldehyd das 1,3-Bis[[2-(dimethylamino)-5-methylbenzyliden]amino]-2-äthylimidazoliumchlorid vom Schmelzpunkt 185° (aus Äthanol/Äther).

Beispiel 2

a) 1,0 g (6,2 mMol) 1,3-Diamino-2-äthyl-imidazoliumchlorid wird auf eine mit 30 g Amberlite™ IR45 (Acetat) beladene Säule gebracht, worauf man mit ca. 600 ml Wasser eluiert. Das Eluat wird eingedampft. Man erhält 1,3-Diamino-2-äthyl-imidazoliumacetat mit einem ungefähren Schmelzpunkt von 20–25°.

b) 1,15 g (6,2 mMol) 1,3-Diamino-2-äthyl-imidazoliumacetat werden in 10 ml Eisessig gelöst, worauf man mit 1,85 g (12,4 mMol) 4-Dimethylaminobenzaldehyd versetzt. Man läßt während 16 Stunden bei Raumtemperatur stehen, verdünnt die Lösung mit 30 ml Äthanol und fällt das Produkt durch Zugabe von Äther aus. Nach Umkristallisieren aus Äthanol/Äther erhält man 1,3-Bis[[(p-dimethylamino)benzyliden]amino-2-äthylimidazoliumacetat vom Schmelzpunkt 166–167° (Zersetzung).

Beispiel 3

Man gibt zu einer Lösung von 0,69 g (3,7 mMol) 1,3-Diamino-2-äthyl-imidazoliumacetat in 10 ml Eisessig 1,95 g (7,4 mMol) p-[4-(Äthoxycarbonyl)-1-piperazinyl]benzaldehyd, läßt während 18 Stunden bei Raumtemperatur stehen, verdünnt dann mit 30 ml Äthanol und kristallisiert das Produkt durch Zugabe von Äther aus. Man erhält 1,3-Bis[[p-(4-(äthoxycarbonyl)-1-piperazinyl]benzyliden]amino]-2-äthyl-imidazoliumacetat-diacetat vom Schmelzpunkt 187–188°.

Beispiel 4

a) 1,5 g (9,25 mMol) 1,3-Diamino-2-äthyl-imidazoliumchlorid werden auf eine mit 30 g Amberlite™ IR45 (Propionat) beladene Säule gebracht, worauf man mit ca. 600 ml Wasser eluiert. Das Eluat wird eingedampft. Man erhält 1,3-Diamino-2-äthyl-imidazoliumpropionat mit einem ungefähren Schmelzpunkt von 20–25°.

b) Zu einer Lösung von 1,85 g (9,25 mMol) 1,3-Diamino-2-äthyl-imidazoliumpropionat in 10 ml Propionsäure gibt man 3,88 g (18,5 mMol) 4-Dimethylamino-3,5-dimethoxybenzaldehyd. Man läßt während 16 Stunden bei Raumtemperatur stehen und kristallisiert das Produkt durch Zugabe von Äther und Petroläther aus. Nach Umkristallisieren aus Äther erhält man 1,3-Bis[[4-(dimethylamino)-3,5-dimethoxybenzyliden]-amino]-2-äthylimidazoliumpropionat vom Schmelzpunkt 138–139°.

Beispiel 5

a) 324 mg (2 mMol) 1,3-Diamino-2-äthyl-imidazoliumchlorid werden auf eine mit 30 g Amberlite™ IR45 (Citrat) beladene Säule gebracht, worauf man mit ca. 500 ml Wasser eluiert. Nach Eindampfung des Eluates erhält man 1,3-Diamino-2-äthyl-imidazoliumcitrat als gelbes Öl.

b) 910 mg (1,5 mMol) 1,3-Diamino-2-äthyl-imidazoliumcitrat werden in 20 ml 2-Propanol gelöst, worauf man mit 630 mg (3 mMol) 4-Dimethylamino-3,5-dimethoxybenzaldehyd versetzt, während 2 Tagen bei Raumtemperatur stehen läßt und die gelbe Lösung dann einengt. Durch Zugabe von Petroläther wird das

Produkt auskristallisiert. Nach Umkristallisieren aus Äthanol/Petroläther erhält man 1,3-Bis[[4-(dimethylamino)-3,5-dimethoxybenzyliden]amino]-2-äthylimidazoliumcitrat vom Schmelzpunkt 140–144°.

Beispiel 6

a) Zu einer Lösung von 3,24 g (20 mMol) 1,3-Diamino-2-äthylimidazoliumchlorid in 180 ml Eisessig werden 2,98 g (20 mMol) p-Dimethylaminobenzaldehyd gegeben. Man rührt während 50 Stunden bei Raumtemperatur, filtriert dann das ausgefallene 1,3-Bis[[p-(dimethylamino)benzyliden]amino]-2-äthylimidazoliumchlorid ab und wäscht es mit Äthanol. Die Mutterlauge wird eingedampft, und der Rückstand wird auf eine mit 200 g Kieselgel (Korngröße: 0,063–0,200 mm) beladene Säule gebracht. Durch Eluieren mit Methylenchlorid/Methanol (9:1 v/v) erhält man eine weitere Portion an 1,3-Bis[[p-(dimethylamino)-benzyliden]amino]-2-äthyl-imidazoliumchlorid.

Durch Eluieren mit Methylenchlorid/Methanol (3:2 v/v) und anschließendes Umkristallisieren des erhaltenen Materials aus Äthanol/Äther erhält man 3-Amino-1-[[p-(dimethylamino)benzyliden]amino]-2-äthylimidazoliumchlorid vom Schmelzpunkt 207–208° (Zersetzung).

Beispiel 7

Zu einer Lösung von 1,19 g (4 mMol) 3-Amino-1-[[p-(dimethylamino)benzyliden]amino]-2-äthylimidazoliumchlorid in 10 ml Eisessig gibt man 0,61 g (4 mMol) p-Nitrobenzaldehyd, rührt während 16 Stunden bei Raumtemperatur und fällt das Produkt durch Zugabe von Äther aus. Das Produkt wird dann abfiltriert und aus Äthanol umkristallisiert. Man erhält 1-[[p-(Dimethylamino)benzyliden]amino]-2-äthyl-3-[(p-nitrobenzyliden)amino]imidazoliumchlorid vom Schmelzpunkt 250°.

Beispiel 8

Zu einer Lösung von 1,0 g (4,18 mMol) 3-Amino-1-[[p-(dimethylamino)benzyliden]amino]-2-äthylimidazoliumchlorid in 40 ml Eisessig gibt man 0,58 g (4,14 mMol) p-Chlorbenzaldehyd und läßt während 24 Stunden bei Raumtemperatur stehen. Das Produkt wird dann abfiltriert, mit Äther gewaschen und aus Äthanol umkristallisiert. Man erhält 3-[p-(Chlorbenzyliden)amino]-1-[[p-(dimethylamino)benzyliden]-amino]-2-äthylimidazoliumchlorid vom Schmelzpunkt 246° (Zersetzung).

Beispiel 9

a) Zu einer Lösung von 1,99 g (10 mMol) 1-Benzylidenamino-2-äthylimidazol in 10 ml Acetonitril gibt man 2,16 g (10 mMol) 3,4,5-Trimethoxybenzylchlorid und erhitzt während 28 Stunden unter Rückfluß. Das auskristallisierte Produkt wird abfiltriert und mit Äther gewaschen. Man erhält 1-Benzylidenamino-2-äthyl-3-(3,4,5-trimethoxybenzyl)imidazoliumchlorid vom Schmelzpunkt 205°.

b) 3,1 g (7,45 mMol) 1-Benzylidenamino-2-äthyl-3-(3,4,5-trimethoxybenzyl)imidazoliumchlorid werden in 70 ml Wasser gelöst, worauf man mit 8 ml 25-proz. Salzsäure versetzt. Der entstehende Benzaldehyd wird mittels Wasserdampfdestillation entfernt. Nach dem Eindampfen wird das Produkt aus Äthanol/Äther umkristallisiert. Man erhält 1-Amino-2-äthyl-3-(3,4,5-trimethoxybenzyl)imidazoliumchlorid vom Schmelzpunkt 174°.

c) 1,9 g (5,8 mMol) 1-Amino-2-äthyl-3-(3,4,5-trimethoxybenzyl)imidazoliumchlorid werden in 38 ml Eisessig gelöst, worauf man mit 0,86 g (5,8 mMol) p-Dimethylaminobenzaldehyd versetzt, und während 52 Stunden bei Raumtemperatur rührt und die Lösung eindampft. Das Produkt wird aus Äthanol/Äther umkristallisiert. Man erhält 2-Äthyl-1-[[p-(dimethylamino)benzyliden]amino]-3-(3,4,5-trimethoxybenzyl)imidazoliumchlorid vom Schmelzpunkt 211°.

Beispiel 10

a) Zu einer Lösung von 1,99 g (10 mMol) 1-Benzylidenamino-2-äthylimidazol in 80 ml Äthylenchlorid werden 2,23 g (10 mMol) 4-Methoxyphenacylbromid gegeben. Man rührt während 3 Stunden bei 60°, filtriert das auskristallisierte Produkt ab und wäscht es mit Äther. Man erhält 1-(Benzylidenamino)-2-äthyl-3-(p-methoxyphenacyl)imidazoliumbromid vom Schmelzpunkt 221–222°.

b) Ein Gemisch aus 0,86 g (2 mMol) 1-Benzylidenamino)-2-äthyl-3-(p-methoxyphenacyl)imidazoliumbromid und 10 ml 5-proz. Bromwasserstoffsäure wird erwärmt, wobei der entstehende Benzaldehyd mittels Wasserdampfdestillation entfernt wird. Das nach dem Eindampfen erhaltene Produkt wird aus Äthanol/Äther umkristallisiert. Man erhält 1-Amino-2-äthyl-3-(p-methoxyphenacyl)-2-äthylimidazoliumbromid vom Schmelzpunkt 213–215°.

c) Zu einer Lösung von 2,7 g (7,9 mMol) 1-Amino-2-äthyl-3-(p-methoxyphenacyl)-2-äthylimidazoliumbromid in 40 ml Eisessig werden 1,18 g (7,9 mMol) p-Dimethylaminobenzaldehyd gegeben. Man rührt während 4 Tagen bei Raumtemperatur, kristallisiert das Produkt durch Zugabe von Äther aus und filtriert es ab. Es wird mit Wasser gewaschen, bei Raumtemperatur getrocknet und aus Äthanol umkristallisiert.

Man erhält 1-[[p-(Dimethylamino)benzyliden]amino]-2-äthyl-3-(p-methoxyphenacyl)imidazoliumbromid vom Schmelzpunkt 244–246°.

Beispiel 11

a) Zu einer Lösung von 1,79 g (10 mMol) 1-Brom-3,3-dimethyl-2-butanon in 30 ml Äthylenchlorid gibt man 1,99 g (10 mMol) 1-(Benzylidenamino)-2-äthyl-imidazol und rührt dann während 5 Stunden bei 60°. Das auskristallisierte Produkt wird abfiltriert und mit Äther gewaschen. Man erhält 1-(Benzylidenamino)-2-äthyl-3-(pivaloylmethyl)imidazoliumbromid vom Schmelzpunkt 219–221°.

b) 756 mg (2 mMol) 1-(Benzylidenamino)-2-äthyl-3-(pivaloylmethyl)imidazoliumbromid werden in einem Gemisch aus 5 ml Wasser und 1 ml 48-proz. Bromwasserstoffsäure solange einer Wasserdampfdestillation unterworfen, bis kein Benzaldehyd mehr entweicht. Hierauf wird eingedampft, und der Rückstand wird das Äthanol/Äther umkristallisiert. Man erhält 1-Amino-2-äthyl-3-(3,3-dimethyl-2-oxobutyl)imidazoliumbromid vom Schmelzpunkt 222–223°.

c) 2,38 g (8,2 mMol) 1-Amino-2-äthyl-3-(3,3-dimethyl-2-oxobutyl)imidazoliumbromid werden in 30 ml Eisessig gelöst, worauf man mit 1,22 g (8,2 mMol) p-Dimethylaminobenzaldehyd versetzt. Das Produkt wird nach Rühren bei Raumtemperatur während 48 Stunden durch Zugabe von Äther umkristallisiert, an 20 g Kieselgel (Korngröße 0,063–0,200 mm) unter Eluieren mit Methylenchlorid/Methanol (19:1 v/v) chromatographiert und aus Äthanol/Äther umkristallisiert. Man erhält 1-[[4-(Dimethylamino)benzyliden]-amino]-2-äthyl-3-(pivaloylmethyl)imidazoliumbromid vom Schmelzpunkt 188–189°.

Beispiel 12

a) Zu einer Lösung von 2,22 g (15 mMol) 1-Amino-2-äthylimidazol-Hydrochlorid in 40 ml Eisessig werden 2,24 g (15 mMol) p-Dimethylaminobenzaldehyd gegeben. Nach Stehenlassen während 24 Stunden bei Raumtemperatur wird das Produkt durch Zugabe von Äther auskristallisiert und anschließend aus Äthanol/Äther umkristallisiert. Man erhält 1-[[p-(Dimethylamino)benzyliden]amino]-2-äthylimidazol Hydrochlorid vom Schmelzpunkt 226–228°.

b) Aus 7,3 g (26 mMol) 1-[[p-(Dimethylamino)benzyliden]amino]-2-äthylimidazol-hydrochlorid wird mit gesättigter Natriumhydrogencarbonatlösung die Base freigesetzt. Diese wird in Methylenchlorid aufgenommen. Man trocknet die Lösung über Natriumsulfat und engt ein. Man erhält 1-[[p-(Dimethylamino)-benzyliden]amino]-2-äthylimidazol vom Schmelzpunkt 120–121°.

c) 1,82 g (7,5 mMol) 1-[[p-(Dimethylamino)benzyliden]amino]-2-äthylimidazol werden in 50 ml Äthylenchlorid gelöst, worauf man mit 1,95 g (10 mMol) Bromessigsäure-t-butylester versetzt. Nach Stehenlassen während 18 Stunden bei Raumtemperatur wird die Lösung eingeengt, und der Rückstand wird aus Äthanol/Äther umkristallisiert. Man erhält 3-[(t-Butoxycarbonyl)methyl]-1-p-[[(dimethylamino)-benzyliden]amino]-2-äthylimidazoliumbromid vom Schmelzpunkt 192° (Zersetzung).

Beispiel 13

a) 1,44 g (6 mMol) 1-[[p-(Dimethylamino)benzyliden]amino]-2-äthylimidazol werden in 20 ml Methylenchlorid gelöst. Man versetzt mit 1,72 g (6 mMol) 3,4,5-Trimethoxyphenacylbromid und läßt während 46 Stunden bei Raumtemperatur stehen. Das Produkt wird durch Zugabe von Äther auskristallisiert und aus Äthanol umkristallisiert. Man erhält 1-[[p-(Dimethylamino)benzyliden]amino]-2-äthyl-3-(3,4,5-trimethoxyphenacyl)imidazoliumbromid vom Schmelzpunkt 232° (Zersetzung).

b) In analoger Weise erhält man aus 1-[[p-(Dimethylamino)benzyliden]amino]-2-isopropylimidazol und 1-Brom-pinakolin das 3-[[p-(Dimethylamino)benzyliden]amino]-2-isopropyl-1-(pivaloylmethyl)imidazoliumbromid vom Schmelzpunkt 236–237° (Zers.; aus Acetonitril/Äther).

Beispiel 14

Eine Lösung von 2,42 g (10 mMol) 1-[[p-(Dimethylamino)benzyliden]amino]-2-äthylimidazol wird mit 2,42 g (10 mMol) p-Dimethylaminophenacylbromid versetzt. Nach Rühren während 30 Minuten bei 40° wird das Produkt durch Zugabe von Äther auskristallisiert und aus Methylenchlorid/Äther umkristallisiert. Man erhält 3-[p-(Dimethylamino)phenacyl]-1-[[p-(dimethylamino)benzyliden]amino]-2-äthylimidazoliumbromid vom Schmelzpunkt 247°.

Beispiel 15

a) 25 g (0,23 Mol) 2-Propylimidazol werden in 70 ml Wasser suspendiert, worauf man innert 20 Minuten unter Rühren mit einer bei 0° hergestellten Lösung von 25 g (0,22 Mol) Natriumhydrogencarbonat in 150 ml Wasser versetzt. Nach Rühren während 20 Stunden bei Raumtemperatur säuert man mit 65 ml 2N-Salzsäure an, gibt eine Lösung von 15,5 g (0,15 Mol) Benzaldehyd in 50 ml Äther hinzu und rührt während 6 Stunden bei Raumtemperatur. Es bilden sich weiße Kristalle, die abfiltriert und in Methylenchlorid gelöst werden. Die Mutterlauge wird wie unten beschrieben weiterverarbeitet. Die Lösung wird über Natriumsulfat getrocknet und eingeengt, worauf man den Rückstand durch Zugabe von Äther auskristallisiert. Nach Umkristallisieren aus Äthanol erhält man 1,3-Bis[[benzyliden]amino]-2-propyl-imidazolium-benzaldoxim-O-sulfonat vom Schmelzpunkt 143–145°.

b) Die obige Mutterlauge wird mit Äther gewaschen, mit 60 ml 3N-Natronlauge versetzt und mit Methylenchlorid extrahiert. Nach Trocknen des Auszuges über Natriumsulfat erhält man ein rötliches Öl, das man Kieselgel (Korngröße 0,063–0,2 mm) unter Eluieren mit Methylenchlorid/Methanol (99:1 v/v) chromatographiert. Man erhält 1-(Benzyliden)amino-2-propyl-imidazol vom Schmelzpunkt 61–62°.

c) In analoger Weise erhält man aus 2-Butylimidazol, Hydroxylamin-O-sulfonsäure und Benzaldehyd das 1,3-Bis[[benzyliden]amino]-2-butyl-imidazoliumbenzaldoxim-O-sulfonat vom Schmelzpunkt 137–140° (nicht umkristallisiert) und

d) 1-(Benzyliden)amino-2-butyl-imidazol vom Schmelzpunkt 60–61° (aus Pentan).

e) 18,6 g (36 mMol) 1,3-Bis[[benzyliden]amino]-2-propyl-imidazolium-benzaldoxim-O-sulfat werden in 100 ml Wasser und 70 ml 2N-Salzsäure suspendiert und auf dem Dampfbad einer Wasserdampfdestillation unterworfen, bis kein Benzaldehyd mehr entweicht. Die Lösung wird eingedampft, und der Rückstand wird aus Äthanol/Äther kristallisiert. Das Rohprodukt wird auf eine mit 200 ml Ionenaustauscher Amberlite™ IRA 400 (Chlorid) beladene Säule gebracht, worauf man mit Wasser eluiert. Das Eluat wird eingedampft, und der Rückstand wird aus Äthanol/Äther umkristallisiert. Man erhält 1,3-Diamino-2-propylimidazoliumchlorid vom Schmelzpunkt 197–199°.

f) In analoger Weise erhält man aus 1,3-Bis[[benzyliden]amino]-2-butyl-imidazoliumbenzaldoxim-O-sulfonat das 1,3-Diamino-2-butyl-imidazoliumchlorid vom Schmelzpunkt 146–147° (aus Äthanol/Äther).

g) Zu einer Lösung von 0,88 g (5 mMol) 1,3-Diamino-2-propyl-imidazoliumchlorid in 15 ml Eisessig werden 1,49 g (10 mMol) 4-Dimethylamino-benzaldehyd gegeben. Nach Stehenlassen bei Raumtemperatur während 2 Tagen wird das Produkt durch Zugabe von Äther auskristallisiert und dann aus Äthanol umkristallisiert. Man erhält 1,3-Bis[[p-(dimethylamino)benzyliden]amino]-2-propyl-imidazoliumchlorid vom Schmelzpunkt 252°.

In analoger Weise erhält man:

h) Aus 1,3-Diamino-2-butyl-imidazoliumchlorid und 4-Dimethylamino-benzaldehyde das 2-Butyl-1,3-bis[[p-(dimethylamino)benzyliden]amino]imidazoliumchlorid vom Schmelzpunkt 240° (aus Äthanol) (Zersetzung);

i) aus 1,3-Diamino-2-isopropylimidazoliumchlorid das 1,3-Bis[[p-(dimethylamino)benzyliden]amino]-2-isopropylimidazoliumchlorid vom Schmelzpunkt 241°.

Beispiel 16

Zu einer Lösung von 0,60 g (3,4 mMol) 1,3-Diamino-2-propyl-imidazoliumchlorid in 5 ml Eisessig werden 1,43 g (6,8 mMol) 4-Dimethylamino-3,5-dimethoxy-benzaldehyd gegeben. Nach Stehenlassen während 16 Stunden wird das Produkt durch Zugabe von Äther auskristallisiert und dann aus Äthanol umkristallisiert. Man erhält 1,3-Bis[[4-(dimethylamino)-3,5-dimethoxybenzyliden]amino]-2-propylimidazoliumchlorid vom Schmelzpunkt 218–219°.

Beispiel 17

a) zu einer Lösung von 7,26 g (88 mMol) 2-Methylimidazol in 30 ml Wasser wird eine bei 0° hergestellte Lösung von 10 g (88 mMol) Hydroxylamin-O-sulfonsäure und 7,4 g (88 mMol) Natriumhydrogencarbonat in 60 ml Wasser getropft. Die Temperatur steigt dabei auf 35° an. Nach Rühren während 16 Stunden wird mit 26 ml 2N-Salzsäure angesäuert. Eine Lösung von 6,26 g (59 mMol) Benzaldehyd in 20 ml Äther wird dazugegeben, worauf man noch während 6 Stunden rührt. Das ausgefallene Produkt wird abfiltriert und aus Methanol/Äther umkristallisiert. Man erhält 1,3-Bis(benzylidenamino)-2-methylimidazoliumbenzaldoxim-O-sulfonat vom Schmelzpunkt 249–250°.

b) 19,2 g (39 mMol) 1,3-Bis(benzylidenamino)-2-methylimidazoliumbenzaldoxim-O-sulfont werden in 100 ml Wasser und 70 ml 2N-Salzsäure einer Wasserdampfdestillation unterworfen, bis kein Benzaldehyd mehr entsteht. Es wird eingedampft, und der Rückstand wird auf eine mit 200 ml Ionenaustauscher Amberlite™ IRA 400 (Chlorid) beladene Säule gebracht. Man eluiert mit ca. 1 Liter Wasser, dampft das Eluat ein und kristallisiert das erhaltene Material aus Äthanol/Methylenchlorid um. Man erhält 1,3-Diamino-2-methyl-imidazoliumchlorid vom Schmelzpunkt 225–227°.

c) 1,0 g (6,76 mMol) 1,3-Diamino-2-methyl-imidazoliumchlorid werden auf eine mit 30 g Amberlite™ IR 45 (Acetat) beladene Säule gebracht, worauf man mit Wasser eluiert. Nach Eindampfen der Lösung erhält man 1,3-Diamino-2-methylimidazoliumacetat als dickflüssiges Öl, das sich allmählich verfestigt.

d) Zu einer Lösung von 1,1 g (6,4 mMol) 1,3-Diamino-2-methylimidazoliumacetat in 11 ml Eisessig werden 1,9 g (12,8 mMol) 4-Dimethylamino-benzaldehyd gegeben. Nach Stehenlassen bei Raumtemperatur während 16 Stunden werden 50 ml Äthanol zugegeben, und das Produkt wird durch Zusatz von Äther auskristallisiert. Nach Umkristallisieren aus Äthanol/Äther erhält man 1,3-Bis[[p-dimethylamino)benzyliden]-amino]-2-methylimidazoliumacetat vom Schmelzpunkt 166–167° (Zersetzung).

In analoger Weise erhält man:

e) Mit 4-Pyrrolidinobenzaldehyd das 1,3-Bis[[4-pyrrolidinobenzyliden]amino]-2-methylimidazoliumchlorid vom Schmelzpunkt 258° (aus Äthanol/Äther);
f) mit 3-(Dimethylamino)-5-phenylbenzaldehyd nach Chromatographieren an Kieselgel mit n-Butanol/Wasser/Essigester (4:1:1) das 1,3-Bis[[2-(dimethylamino)-5-phenylbenzyliden]amino]-2-methylimidazoliumchlorid-Hydrochlorid vom Schmelzpunkt 268–270° (aus äthanolischer Salzsäure);
g) mit 5-(Dimethylamino)-2-thiophencarboxaldehyd nach Chromatographieren an Kieselgel mit n-Butanol/Wasser/Essigsäure (4:1:1) das 1,3-Bis[[5-(dimethylamino-2-thienylmethylen]amino]-2-methylimidazoliumchlorid vom Schmelzpunkt 260–262° (aus Äthanol).

Beispiel 18

a) Zu einer Lösung von 1,99 g (10 mMol) 1-(Benzyliden)amino-2-äthylimidazol in 10 ml Acetonitril werden 1,61 g (10 mMol) p-Chlorbenzylchlorid gegeben. Nach Erhitzen unter Rückfluß während 24 Stunden wird das ausgefallene Produkt abfiltriert und aus Äthanol/Äther umkristallisiert. Man erhält 1-(Benzylidenamino)-3-(p-chlorbenzyl)-2-äthylimidazoliumchlorid vom Schmelzpunkt >200°.
b) 500 mg (1,39 mMol) 1-(Benzylidenamino)-3-(p-chlorbenzyl)-2-äthylimidazoliumchlorid werden in 5 ml Wasser und 2 ml 3N-Salzsäure einer Wasserdampfdestillation unterworfen, bis kein Benzaldehyd mehr entweicht. Es wird eingedampft, und der Rückstand wird aus Äthanol/Äther umkristallisiert. Man erhält 1-Amino-3-(p-chlorbenzyl)-2-äthylimidazoliumchlorid vom Schmelzpunkt 223–225°.
c) 2,44 g (9 mMol) 2-(p-Chlorbenzyl)-2-äthylimidazoliumchlorid werden in 25 ml Eisessig gelöst, worauf man mit 1,34 g (9 mMol) 4-Dimethylaminobenzaldehyd versetzt. Nach Rühren bei Raumtemperatur während 24 Stunden wird das Produkt durch Zugabe von Äther auskristallisiert und aus Äthanol/Äther umkristallisiert. Man erhält 3-(p-Chlorbenzyl)-1-[[p-(dimethylamino)benzyliden]amino]-2-äthylimidazoliumchlorid vom Schmelzpunkt 79–81°.

Beispiel 19

a) Zu einer Lösung von 1,99 g (10 mMol) 1-(Benzyliden)amino-2-äthylimidazol in 30 ml Äthylenchlorid werden 2,33 g (10 mMol) p-Chlorphenacylbromid gegeben. Nach Rühren bei 60° während 2 Stunden wird das Produkt abfiltriert und mit Äther gewaschen. Man erhält 1-(Benzylidenamino)-3-(p-chlorphenacyl)-2-äthylimidazoliumbromid vom Schmelzpunkt 236–237°.
b) 864 mg (2 mMol) 1-(Benzylidenamino)-3-(p-chlorphenacyl)-2-äthylimidazoliumbromid werden in 5 ml Wasser und 2 ml 48-proz. Bromwasserstoffsäure einer Wasserdampfdestillation unterworfen, bis kein Benzaldehyd mehr entweicht. Die Lösung wird konzentriert, und das ausgefallene Produkt wird abfiltriert. Man erhält 1-Amino-3-(p-chlorphenacyl)-2-äthylimidazoliumbromid vom Schmelzpunkt 209–210°.
c) Eine Suspension bestehend aus 1,72 g (5 mMol) 1-Amino-3-(p-chlorphenacyl)-2-äthylimidazoliumbromid und 0,74 g (5 mMol) 4-Dimethylaminobenzaldehyd in 15 ml Eisessig wird während 48 Stunden bei Raumtemperatur gerührt, mit Äther versetzt und filtriert. Das abfiltrierte Rohprodukt wird an 30 g Kieselgel (Korngröße 0,063–0,200 mm) unter Eluieren mit Methylenchlorid/Methanol (9:1 v/v) chromatographiert und dann aus Methylenchlorid/Methanol/Äther umkristallisiert. Man erhält 3-(p-Chlorphenacyl)-1-[p-dimethylamino)benzyliden]amino]-2-äthylimidazoliumbromid vom Schmelzpunkt 252–253°.

Beispiel 20

2,2 g 1,3-Diamino-2-methylimidazoliumchlorid und 6,9 g 4-Dimethylamino-3,5-dimethoxy-benzaldehyd werden in 100 ml Eisessig gelöst, worauf man während 48 Stunden bei Raumtemperatur rührt. Der Eisessig wird in Vakuum abdestilliert, und der Rückstand wird in 100 ml Essigester gelöst. Nach Einsetzen der Kristallisation werden weitere 300 ml Essigester dazugegeben, worauf man auf 10° kühlt. Das auskristallisierte Material wird genutscht, mit 100 ml Essigester gewaschen und im Vakuum bei 80° über Kaliumhydroxid getrocknet. Man erhält 1,3-Bis[[4-(dimethylamino)-3,5-dimethoxybenzyliden]amino]-2-methylimidazoliumchlorid vom Zersetzungspunkt 226°.

Beispiel 21

a) 1,3 g (10 mMol) 1-Amino-2-methylimidazol-hydrochlorid und 2,2 g (15 mMol) p-Dimethylaminobenzaldehyd werden in 100 ml Eisessig während 16 Stunden bei Raumtemperatur gerührt. Es wird eingedampft, worauf man noch viermal mit Äthanol versetzt und jeweils wieder eindampft. Der kristalline Rückstand wird mit Äther gewaschen und dann mit gesättigter Natriumhydrogencarbonatlösung versetzt. Man extrahiert mit Methylenchlorid, trocknet den Auszug über Natriumsulfat, dampft ein und kristallisiert das erhaltene Material aus Methylenchlorid/Petroläther um. Man erhält 1-[[p-(Dimethylamino)benzyliden]amino]-2-methylimidazol vom Schmelzpunkt 166–167°.
b) 228 mg (1 mMol) 1-[[p-(Dimethylamino)benzyliden]amino]-2-methylimidazol werden in 20 ml Methylenchlorid (bei einem weiteren Versuch wurde Methylenchlorid/Äthanol als Lösungsmittel verwendet) ge-

löst, worauf man innert 2 Wochen unter Rühren portionenweise mit 466 mg (2 mMol) o-Diphenylphosphinylhydroxylamin versetzt. Es wird eingedampft; der Rückstand wird in 200 ml Wasser aufgenommen und auf eine mit 10 ml Ionenaustauscher Amberlite™ IRA 400 (Chlorid) beladene Säule gebracht. Man eluiert mit Wasser, dampft das Eluat ein und chromatographiert das erhaltene Material an 20 g Kieselgel (Korngröße 0,063–0,2 mm) mit Methylenchlorid/Methanol (7:3 v/v). Durch Umkristallisieren aus Äthanol/Äther erhält man 1-Amino-3-[[p-(dimethylamino)benzyliden]amino-2-methylimidazoliumchlorid vom Schmelzpunkt 230–231°.

c) 56 mg (0,2 mMol) 1-Amino-3-[[p-(dimethylamino)benzyliden]amino]-2-methylimidazoliumchlorid und 30 mg (0,2 mMol) p-Nitrobenzaldehyd werden in 5 ml Eisessig gelöst. Nach 2 Tagen wird eingdampft, und der Rückstand wird an 5 g Kieselgel (Korngröße 0,063–0,200 mm) unter Eluieren mit Chloroform/Methanol (87:13) chromatographiert und aus Methylenchlorid/Petroläther umkristallisiert. Man erhält 1-[[p-(Dimethylamino)benzyliden]-2-methyl-3-[[p-nitrobenzyliden]amino]-imidazoliumchlorid vom Schmelzpunkt 252° (Zersetzung).

Beispiel 22

a) 7,92 g (171 mMol) Natriumhydrid (55–60-proz. Dispersion in Öl) werden mit absolutem Tetrahydrofuran gewaschen. Eine Lösung von 13,2 g (83 mMol) 2-Methyl-4-phenylimidazol in 356 ml N-Methylpyrrolidon wird zugetropft bei 0°. Anschließend wird bei Raumtemperatur gerührt, bis keine Gasentwicklung mehr zu beobachten ist. Man gibt 39,6 g (171 mMol) O-Diphenylphosphinylhydroxylamin portionenweise dazu und rührt noch 18 Stunden. 400 ml Wasser werden zugefügt. Man rührt 1 Stunde und extrahiert viermal mit je 1300 ml Methylenchlorid. Der Extrakt wird über Natriumsulfat getrocknet und eingedampft. Der Rückstand wird auf eine mit 700 g Kieselgel (Korngröße 0,063–0,200 mm) beladene Säule gebracht und das Produkt wird mit Methylenchlorid/Äthanol (98:2) eluiert. Das nach Einengen erhaltene Öl (N-Amino-2-methyl-4-phenylimidazol) wird in 1150 ml Methylenchlorid gelöst und mit 13,94 g (60 mMol) O-Diphenylphosphinylhydroxylamin versetzt. Nach drei Tagen Rühren gibt man nochmals 13,94 g (60 mMol) O-Diphenylphosphinylhydroxylamin dazu und rührt weitere 24 Stunden. Man engt ein und bringt den Rückstand auf eine mit Amberlite™ IRA 400 (Chlorid) beladene Säule. Es wird mit Wasser eluiert. Die wässrige Lösung wird eingedampft, und der Rückstand wird auf eine mit 300 g Kieselgel (Korngröße 0,063–0,200 mm) beladene Säule gebracht. Das Produkt wird mit Methylenchlorid/Äthanol (1:9) eluiert. Aus dem Eluat erhält man 1,3-Diamino-2-methyl-4-phenylimidazoliumchlorid vom Schmelzpunkt 162–163°.

b) 3,6 g (16 mMol) des Chlorides werden in 60 ml Eisessig gelöst. 4,78 g (32 mMol) p-Dimethylaminobenzaldehyd werden zugegeben. Nach 20 Stunden Rühren gibt man 10 ml Äther dazu und rührt während 24 Stunden weiter. Man engt ein und bringt den Rückstand auf eine mit 280 g Kieselgel (Korngröße 0,063–0,200 mm) beladene Säule. Das Produkt wird mit Methylenchlorid eluiert und aus Äthanol/Äther kristallisiert. Man erhält 1,3-Bis[[p-(dimethylamino)benzyliden]amino]-2-methyl-4-phenylimidazoliumchlorid vom Schmelzpunkt 254°.

In analoger Weise erhält man:

c) 1,3-Bis[[p-(dimethylamino)benzyliden]amino]-2-äthyl-4-methyl-imidazoliumchlorid vom Schmelzpunkt 218–219°;

d) 1,3-Bis[(p-dimethylamino)benzyliden]-2-äthylbenzimidazoliumchlorid vom Schmelzpunkt 152–153°.

Beispiel 23

a) 86,0 g (767 mMol) 4-Hydroxymethyl-2-methylimidazol werden in 240 ml Wasser gelöst. Dazu tropft man innert 15 Minuten eine frisch hergestellte Lösung von 89,35 g (790 mMol) Hydroxylamin-O-sulfonsäure und 66,4 g (790 mMol) Natriumbicarbonat in 390 ml Wasser. Es wird 20 Stunden bei Raumtemperatur gerührt. Man säuert mit 200 ml 2N-Salzsäure an und gibt 55,4 g (522 mMol) Benzaldehyd gelöst in 280 ml Äther dazu. Man rührt 6 Stunden, filtriert das ausfallende Produkt ab, wäscht es mit Wasser und Äther und trocknet es bei 40° im Hochvakuum. Man erhält 1,3-Bis[[benzyliden]amino]-4-hydroxymethyl-2-methylimidazoliumbenzaldoxim-O-sulfonat vom Schmelzpunkt 161–163° (Rohprodukt).

b) Dieses Produkt wird in 200 ml Wasser und 100 ml 3N-Salzsäure suspendiert und auf dem Dampfbad einer Wasserdampfdestillation unterworfen, bis kein Benzaldehyd mehr entweicht. Die Lösung wird eingedampft und der Rückstand wird auf eine mit 100 ml Ionenaustauscher Amberlite™ IRA 400 (Chlorid) beladene Säule gebracht, worauf man mit Wasser eluiert. Das Eluat wird eingedampft und der Rückstand wird aus Äthanol/Äther kristallisiert. Man erhält 1,3-Diamino-4-(hydroxymethyl)-2-methylimidazoliumchlorid vom Schmelzpunkt 129–130°. Aus der Mutterlauge erhält man noch eine weitere Portion vom Schmelzpunkt 125–126°.

c) 0,89 g (5 mMol) 1,3-Diamino-4-(hydroxymethyl)-2-methylimidazoliumchlorid und 1,49 g (10 mMol) 4-Dimethylaminobenzaldehyd werden in 20 ml Eisessig gelöst. Nach 22 Stunden wird das ausgefallene Produkt abfiltriert und aus Äthanol umkristallisiert. Man erhält 1,3-Bis[[p-(dimethylamino)benzyliden]amino]-4-(hydroxymethyl)-2-methylimidazoliumchlorid vom Schmelzpunkt 237° (Zers.).

Beispiel 24

a) 3,8 g (21,3 mMol) 1,3-Diamino-4-(hydroxymethyl)-2-methylimidazoliumchlorid werden in 200 ml Dichlormethan aufgenommen und mit 2,56 g (21,3 mMol) Thionylchlorid versetzt. Man rührt 3 Tage bei Raumtemperatur, filtriert das Produkt ab, wäscht es mit Dichlormethan und trocknet es im Hochvakuum bei 40°. Man erhält 1,3-Diamino-4-(chlormethyl)-2-methylimidazoliumchlorid vom Schmelzpunkt 163–165°.

b) 0,985 g (5 mMol) 1,3-Diamino-4-(chlormethyl)-2-methylimidazoliumchlorid und 7,45 g (50 mMol) 4-Dimethylaminobenzaldehyd werden in 250 ml Eisessig gelöst. Man läßt die Lösung 36 Stunden bei Raumtemperatur stehen und dampft ein. Der Rückstand wird auf eine mit 150 g Kieselgel (Korngröße 0,063–0,2 mm) beladene Säule gebracht, und das Produkt wird mit Chloroform/Methanol (9:1) eluiert. Das Eluat wird eingedampft und das Produkt wird aus Äthanol/Äther kristallisiert. Man erhält 1,3-Bis[[p-(dimethylamino)benzyliden]amino]-4-chlormethyl)-2-methylimidazoliumchlorid vom Schmelzpunkt 188° (Zers.).

Beispiel 25

a) Eine Lösung von 3,24 g (20 mMol) 1,3-Diamino-2-äthylimidazoliumchlorid und 16,5 g (100 mMol) p-Nitroacetophenon in 150 ml Eisessig läßt man 2 Tage stehen, engt ein und kristallisiert aus Äthanol/Äther. Die Kristalle werden auf eine mit 50 g Kieselgel beladene Säule gebracht. Das mit Methylenchlorid/Methanol (7:3) eluierte Material wird aus Äthanol umkristallisiert. Man erhält 1-Amino-2-äthyl-3-[(α-methyl-p-nitrobenzyliden)amino] imidazoliumchlorid vom Schmelzpunkt 201–203°.

b) Eine Lösung von 1,15 g (3,7 mMol) 1-Amino-2-äthyl-3-[(α-methyl-p-nitrobenzyliden)amino] imidazoliumchlorid und 0,55 g (3,7 mMol) p-Dimethylamino-benzaldehyd in 25 ml Eisessig rührt man während 18 Stunden. Durch Zugabe von Äther wird das Produkt auskristallisiert. Es wird aus Äthanol/Äther umkristallisiert. Man erhält 3-[[p-(Dimethylamino)benzyliden]amino]-2-äthyl-1-[(α-methyl-p-nitrobenzyliden)-amino] imidazoliumchlorid vom Schmelzpunkt 233°.

Beispiel 26

a) 380 g (2 mMol) Triäthyloxoniumtetrafluoroborat werden in 15 ml Methylenchlorid gelöst. Bei –15° werden 326 mg (2 mMol) p-Dimethylaminoacetophenon zugegeben. Nach 5 Minuten gibt man bei der gleichen Temperatur 162 mg (1 mMol) 1,3-Diamino-2-äthyl-imidazoliumchlorid dazu, rührt 1 Stunde bei –10° und 2 Stunden bei 0°. Nach Zugabe von wenig Methanol wird eingedampft und der Rückstand wird auf eine mit 10 g Kieselgel beladene Säule gebracht. Das Produkt wird mit Chloroform/Methanol (87:13, Gew.-Teile) eluiert und mit Äther kristallisiert. Das Kristallisat wird in 80 ml Wasser gelöst, auf eine mit Ionenaustauscher Amberlite™ IRA 400 (Chlorid) beladene Säule gebracht, worauf man mit Wasser eluiert. Das Eluat wird eingedampft und der Rückstand wird aus Äthanol/Äther kristallisiert. Man erhält 3-Amino-1-[[p-(dimethylamino)-α-methylbenzyliden]amino]-2-äthylimidazoliumchlorid vom Schmelzpunkt 223–225°.

b) 435 mg (1,41 mMol) 3-Amino-1-[[p-dimethylamino)-α-methylbenzyliden]amino]-2-äthylimidazoliumchlorid und 210 mg (1,41 mMol) p-Dimethylaminobenzaldehyd werden in 30 ml Eisessig gelöst. Nach 2 Tagen wird die Lösung eingedampft und der Rückstand wird auf eine mit 50 g Kieselgel geladene Säule gebracht. Das Produkt wird mit Dichlormethan/Methanol (9:1) eluiert und aus Äthanol/Äther kristallisiert. Man erhält 3-[[p-(Dimethylamino)benzyliden]amino]-1-[[p-(dimethylamio)-α-methylbenzyliden]amino]-2-äthylimidazoliumchlorid vom Schmelzpunkt >202° (Zersetzung).

Beispiel A

1,3-Bis[[(p-dimethylamino)benzyliden]amino]-2-äthylimidazoliumacetat wird in an sich bekannter Weise als Wirkstoff zur Herstellung von Tabletten nachfolgender Zusammensetzung verwendet:

| | | mg/Tablette |
|---|---|---|
| Wirkstoff | | 100 |
| Lactose | | 192 |
| Maisstärke | | 80 |
| Hydrolysierte Maisstärke | | 20 |
| Calciumstearat | | 8 |
| | Tablettengewicht | 400 mg |

**Patentansprüche für die Vertragsstaaten: BE, CH, DE, FR, GB, IT, LI, NL**

1. Verbindungen der allgemeinen Formel

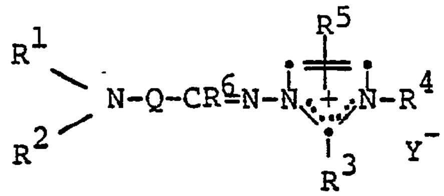

I

worin das Symbol Q eine 1,4-Phenylen- oder 1,4-Naphthylengruppe bedeutet, welche noch durch einen oder zwei Substituenten aus der Gruppe bestehend aus niederem Alkyl und niederem Alkoxy substituiert sein kann, $R^1$ und $R^2$ je Wasserstoff oder niederes Alkyl oder zusammen mit dem Stickstoffatom gegebenenfalls durch eine oder zwei niedere Alkylgruppen substituiertes 4-Morpholinyl, 1-Piperazinyl, 4-(niederes Alkyl)-1-piperazinyl, (4-niederes Alkoxycarbonyl)-1-piperazinyl oder 1-Pyrrolidinyl, $R^3$ niederes Alkyl, $R^4$ die Gruppe $-N{=}CR^6{-}Q{-}NR^1R^2$, $-N{=}CRc{-}Ra$ oder $-CH_2{-}CO{-}Rb$, $R^5$ Wasserstoff, niederes Alkyl, niederes Hydroxyalkyl, niederes Alkoxyalkyl, niederes Halogenalkyl, Phenyl oder einen ankondensierten Benzolring, $R^6$ Wasserstoff oder niederes Alkyl, Ra gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rb niederes Alkyl, niederes Alkoxy oder gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rc Wasserstoff oder niederes Alkyl, die gestrichelte Linie eine zusätzliche Doppelbindung und das Symbol $Y^-$ ein pharmazeutisch annehmbares Anion bedeuten, wobei $R^3$ eine von Methyl verschiedene Bedeutung hat, wenn $R^4$ die Gruppe $-N{=}CH{-}Q{-}NR^1R^2$, $R^1$ und $R^2$ je Methyl, $R^5$ und $R^6$ je Wasserstoff, Q 1,4-Phenylen und Y Chlor bedeuten, und die mit nieder bezeichneten Ausdrücke höchstens 7 Kohlenstoffatome besitzen, und pharmazeutisch annehmbare Säureadditionssalze davon.

2. Verbindungen gemäß Anspruch 1, worin $R^3$ niederes Alkyl, $R^4$ die Gruppe $-N{=}CH{-}Q{-}NR^1R^2$, $-N{=}CH{-}Ra$ oder $-CH_2{-}CO{-}Rb$ und $R^5$ und $R^6$ je Wasserstoff bedeuten.

3. Verbindungen gemäß Anspruch 1 oder 2, worin $R^4$ die Gruppe $-N{=}CR^6{-}Q{-}NR^1R^2$ bedeutet.

4. Verbindungen gemäß Anspruch 3, worin Q 1,4-Phenylen bedeutet.

5. Verbindungen gemäß einem der Ansprüche 1–4, worin $R^1$ und $R^2$ je niederes Alkyl bedeuten.

6. Verbindungen gemäß einem der Ansprüche 1–5, worin $R^3$ niederes Alkyl mit mindestens zwei Kohlenstoffatomen bedeutet.

7. Verbindungen gemäß einem der Ansprüche 1, 2, 4, 5 und 6, worin $R^4$ die Gruppe $-N{=}CRc{-}Ra$ oder $-CH_2{-}CO{-}Rb$ bedeutet.

8. Verbindungen gemäß einem der Ansprüche 1–7, worin $R^5$ Wasserstoff bedeutet.

9. Verbindungen gemäß einem der Ansprüche 1–8, worin $R^6$ und Rc Wasserstoff bedeuten.

10. 3-[p-(Chlorbenzyliden)amino]-1-[[p-(dimethylamino)benzyliden]amino]-2-äthylimidazoliumsalze.

11. 3-[p-(Dimethylamino)phenacyl]-1-[[p-(dimethylamino)benzyliden]amino]-2-äthylimidazoliumsalze.

12. 1,3-Bis[[(p-(dimethylamino)benzyliden]amino]-2-äthylimidazoliumsalze.

13. 1[[p-(Dimethylamino)benzyliden]amino]-2-äthyl-3-[(p-nitrobenzyliden)amino]imidazoliumsalze.

14. 1,3-Bis[[p-(dimethylamino)benzyliden]amino]-2-propylimidazoliumsalze.

15. Verbindungen gemäß einem der Ansprüche 1–14 zur Anwendung als therapeutische Wirkstoffe.

16. Verbindungen gemäß einem der Ansprüche 1–14 zur Anwendung als anthelmintisch wirksame Stoffe.

17. 1,3-Bis[[p-(dimethylamino)benzyliden]-amino]-2-methylimidazoliumchlorid zur Anwendung als therapeutische Wirkstoffe, insbesondere zur Anwendung als anthelmintisch wirksamen Wirkstoff.

18. Verfahren zur Herstellung von Verbindungen gemäß einem der Ansprüche 1–14, dadurch gekennzeichnet, daß man

a) eine Verbindung der allgemeinen Formel

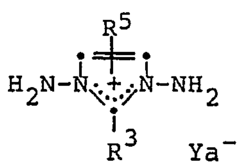

II

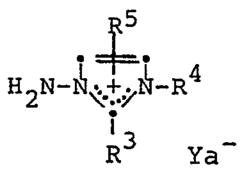

III

oder

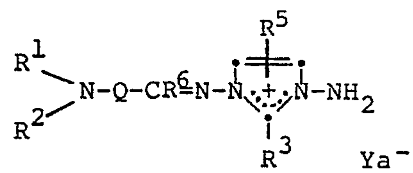

IIIa'

worin $R^1$, $R^2$, $R^3$, $R^4$, $R^5$, $R^6$ und Q die in Anspruch 1 angegebene Bedeutung besitzen, und $Ya^-$ ein Anion bedeutet, mit einer Carbonylverbindung der allgemeinen Formel

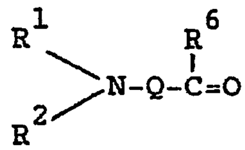

IVa

worin Q, $R^1$, $R^2$ und $R^6$ die in Anspruch 1 angegebene Bedeutung besitzen, oder eine Verbindung der obigen Formel IIIa′ mit einer Carbonylverbindung der allgemeinen Formel

$$Ra–C(Rc)=O \qquad IVb$$

worin Ra und Rc die in Anspruch 1 angegebene Bedeutung besitzen, umsetzt, oder

b) eine Verbindung der allgemeinen Formel

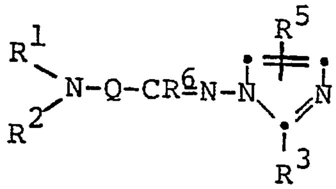

Va'

worin $R^1$, $R^2$, $R^3$, $R^5$, $R^6$ und Q die in Anspruch 1 angegebene Bedeutung besitzen, mit einer Verbindung der allgemeinen Formel

$$R^{41}–X \qquad VI$$

worin $R^{41}$ die Gruppe $–CH_2–CO–Rb$ und X eine Abgangsgruppe bedeuten, und Rb die in Anspruch 1 angegebene Bedeutung besitzt, umsetzt,

c) gegebenenfalls in einer erhaltenden Verbindung das mit $Ya^-$ bezeichnete Anion gegen ein pharmazeutisch annehmbares Anion austauscht und

d) erwünschtenfalls eine erhaltene Verbindung der Formel I in ein pharmazeutisch annehmbares Salz überführt.

19. Verbindungen der allgemeinen Formel

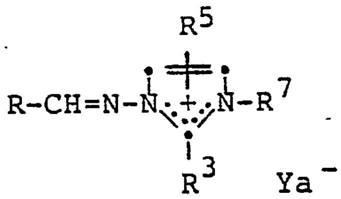

XV

worin $R^7$ die Gruppe $–CH_2–CO–Rb$, R gegebenenfalls durch niederes Alkyl, niederes Alkoxy oder Halogen substituiertes Phenyl und $Ya^-$ ein Anion bedeuten, und $R^3$, $R^5$, Rb, der Ausdruck "nieder" und die gestrichelte Linie die in Anspruch 1 angegebene Bedeutung besitzen.

20. Verbindungen der allgemeinen Formel

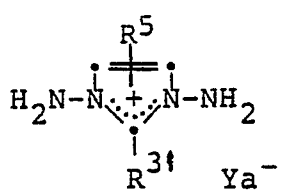

II'

worin $R^{31}$ niederes Alkyl mit mindestens zwei Kohlenstoffatomen und $Ya^-$ ein Anion bedeuten, und $R^5$ und die gestrichelte Linie die in Anspruch 1 angegebene Bedeutung besitzen.

21. Verbindungen der allgemeinen Formel

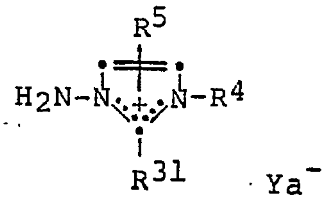

III'

worin $R^{31}$ niederes Alkyl mit mindestens zwei Kohlenstoffatomen und $Ya^-$ ein Anion bedeuten, und $R^4$, $R^5$, der Ausdruck "nieder" und die gestrichelte Linie die in Anspruch 1 angegebene Bedeutung besitzen.

22. Verbindungen der allemeinen Formel

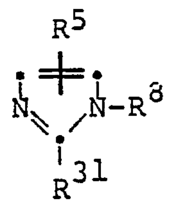

XVI'

worin $R^8$ die Gruppe $-N{=}CR^6-Q-NR^1R^2$ oder $-N{=}CRc-Ra$ und $R^{31}$ niederes Alkyl mit mindestens zwei Kohlenstoffatomen bedeuten und $R^1$, $R^2$, $R^5$, $R^6$, Q, Ra, Rc und der Ausdruck "nieder" die in Anspruch 1 angegebene Bedeutung besitzen.

23. Verwendung von Verbindungen gemäß einem der Ansprüche 19–22 zur Herstellung von Verbindungen gemäß einem der Ansprüche 1–14.

24. Arzneimittel, enthaltend eine Verbindung gemäß einem der Ansprüche 1–17 und ein therapeutisch inertes Trägermaterial.

25. Anthelmintisch wirksame Mittel enthaltend eine Verbindung gemäß einem der Ansprüche 1–17 und ein therapeutisch inertes Trägermaterial.

26. Verwendung von Verbindungen gemäß einem der Ansprüche 1–17 zur Herstellung von anthelmintisch wirksamen Mitteln.

**Patentansprüche für den Vertragsstaat: AT**

1. Verfahren zur Herstellung von Verbindungen der allgemeinen Formel

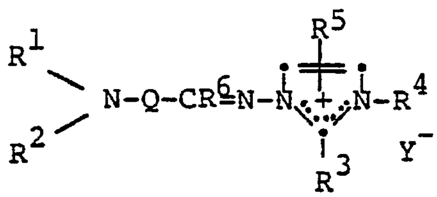

I

worin das Symbol Q eine 1,4-Phenylen- oder 1,4-Napthylengruppe bedeutet, welche noch durch einen oder zwei Substituenten aus der Gruppe bestehend aus niederem Alkyl und niederem Alkoxy substituiert sein kann, $R^1$ und $R^2$ je Wasserstoff oder niederes Alkyl oder zusammen mit dem Stickstoffatom gegebenenfalls durch eine oder zwei niedere Alkylgruppen substituiertes 4-Morpholinyl, 1-Piperazinyl, 4-(niederes Alkyl)-1-piperazinyl, (4-niederes Alkoxycarbonyl)-1-piperazinyl oder 1-Pyrrolidinyl, $R^3$ niederes Alkyl, $R^4$ die Gruppe $-N{=}CR^6-Q-NR^1R^2$, $-N{=}CRc-Ra$ oder $-CH_2-CO-Rb$, $R^5$ Wasserstoff, niederes Alkyl, niederes Hydroxyalkyl, niederes Alkoxyalkyl, niederes Halogenalkyl, Phenyl oder einen ankondensierten Benzolring, $R^6$ Wasserstoff oder niederes Alkyl, Ra gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rb niederes Alkyl, niederes Alkoxy oder gegebenenfalls durch einen oder zwei Substituenten aus der Gruppe bestehend aus niederem Alkyl, niederem Alkoxy, niederem Alkylthio, niederem Alkanoyl, niederem Alkoxycarbonyl, Halogen, Trifluormethyl, Hydroxy, Nitro und Cyano substituiertes Phenyl, Rc Wasserstoff oder niederes Alkyl, die gestrichelte Linie eine zusätzliche Doppelbindung und das Symbol $Y^-$ ein pharmazeutisch annehmbares Anion bedeuten, wobei $R^3$ eine von Methyl verschiedene Bedeutung hat, wenn $R^4$ die Gruppe $-N{=}CH-Q-NR^1R^2$, $R^1$ und $R^2$ je Methyl, $R^5$ und $R^6$ je Wasserstoff, Q 1,4-Phenylen und Y Chlor bedeuten, und die mit nieder bezeichneten Ausdrücke höchstens

7 Kohlenstoffatome besitzen, und von pharmazeutisch annehmbaren Säureadditionssalzen davon, dadurch gekennzeichnet, daß man

a) eine Verbindung der allgemeinen Formel

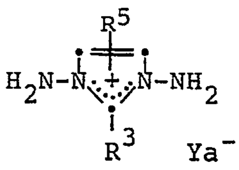

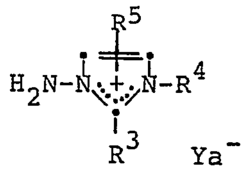

oder

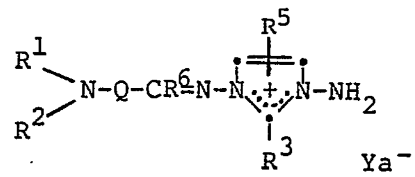

II III IIIa'

worin $R^1$, $R^2$, $R^3$, $R^4$, $R^5$, $R^6$ und Q obige Bedeutung besitzen, und Ya– ein Anion bedeutet, mit einer Carbonylverbindung der allgemeinen Formel

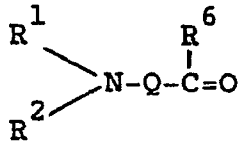

IVa

worin Q, $R^1$, $R^2$ und $R^6$ obige Bedeutung besitzen, oder eine Verbindung der obigen Formel IIIa′ mit einer Carbonylverbindung der allgemeinen Formel

Ra–C(Rc)=O IVb

worin Ra und Rc obige Bedeutung besitzen, umsetzt, oder

b) eine Verbindung der allgemeinen Formel

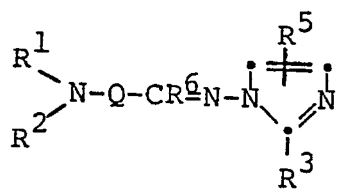

Va'

worin $R^1$, $R^2$, $R^3$, $R^5$, $R^6$ und Q obige Bedeutung besitzen, mit einer Verbindung der allgemeinen Formel

$R^{41}$–X VI

worin $R^{41}$ die Gruppe $-CH_2-CO-Rb$ und X eine Abgangsgruppe bedeuten, und Rb obige Bedeutung besitzt, umsetzt,

c) gegebenenfalls in einer erhaltenen Verbindung das mit $Ya^-$ bezeichnete Anion gegen ein pharmazeutisch annehmbares Anion austauscht und

d) erwünschtenfalls eine erhaltene Verbindung der Formel I in ein pharmazeutisch annehmbares Salz überführt.

2. Verfahren gemäß Anspruch 1, worin $R^3$ niederes Alkyl, $R^4$ die Gruppe $-N=CH-Q-NR^1R^2$, $-N=CH-Ra$ oder $-CH_2-CO-Rb$ und $R^5$ und $R^6$ je Wasserstoff bedeuten.

3. Verfahren gemäß Anspruch 1 oder 2, worin $R^4$ die Gruppe $-N=CR^6-Q-NR^1R^2$ bedeutet.

4. Verfahren gemäß Anspruch 3, worin Q 1,4-Phenylen bedeutet.

5. Verfahren gemäß einem der Ansprüche 1–4, worin $R^1$ und $R^2$ je niederes Alkyl bedeuten.

6. Verfahren gemäß einem der Ansprüche 1–5, worin $R^3$ niederes Alkyl mit mindestens zwei Kohlenstoffatomen bedeutet.

7. Verfahren gemäß einem der Ansprüche 1, 2, 4, 5 und 6, worin $R^4$ die Gruppe $-N=CRc-Ra$ oder $-CH_2-CO-Rb$ bedeutet.

8. Verfahren gemäß einem der Ansprüche 1–7, worin $R^5$ Wasserstoff bedeutet.

9. Verfahren gemäß einem der Ansprüche 1–8, worin $R^6$ und Rc Wasserstoff bedeuten.

10. Verfahren gemäß Anspruch 1, dadurch gekennzeichnet, daß man 3-[p-(Chlorbenzyliden)amino]-1-[[p-(dimethylamino)benzyliden]amino]-2-äthylimidazoliumsalze herstellt.

11. Verfahren gemäß Anspruch 1, dadurch gekennzeichnet, daß man 3-[p-(Dimethylamino)phenacyl]-1-[[p-(dimethylamino)benzyliden]amino]-2-äthylimidazoliumsalze herstellt.
12. Verfahren gemäß Anspruch 1, dadurch gekennzeichnet, daß man 1,3-Bis[(p-(dimethylamino)-benzyliden]amino]-2-äthylimidazoliumsalze herstellt.
13. Verfahren gemäß Anspruch 1, dadurch gekennzeichnet, daß man 1-[[p-(Dimethylamino)benzyliden]-amino]-2-äthyl-3-[(p-nitrobenzyliden)amino]imidazoliumsalze herstellt.
14. Verfahren gemäß Anspruch 1, dadurch gekennzeichnet, daß man 1,3-Bis[[p-(dimethylamino)-benzyliden]amino]-2-propylimidazoliumsalze herstellt.
15. Verwendung von Verbindungen gemäß einem der Ansprüche 1–14 zur Herstellung von anthelmintisch wirksamen Mitteln.
16. Verwendung gemäß Anspruch 1, dadurch gekennzeichnet, daß man 1,3-Bis[[p-(dimethylamino)-benzyliden]amino]-2-methylimidazoliumchlorid zur Herstellung von anthelmintisch wirksamen Mitteln.

**Claims for the Contracting States: BE, CH, DE, FR, GB, IT, LI, NL**

1. Compounds of the general formula

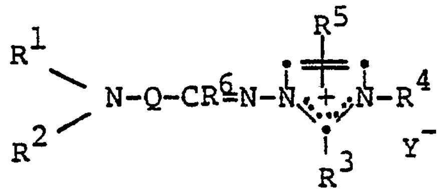

I

wherein the symbol Q signifies a 1,4-phenylene or 1,4-naphthylene group which can be substituted by one or two substitutents from the group consisting of lower alkyl and lower alkoxy, $R^1$ and $R^2$ each signify hydrogen or lower alkyl or together with the nitrogen atom signify 4-morpholinyl, 1-piperazinyl, 4-(lower alkyl)-1-piperazinyl, (4-lower alkoxycarbonyl)-1-piperazinyl or 1-pyrrolidinyl which is optionally substituted by one or two lower alkyl groups, $R^3$ signifies lower alkyl, $R^4$ signifies the group $-N=CR^6-Q-NR^1R^2$, –N=CRc–Ra or $-CH_2-CO-Rb$, $R^5$ signifies hydrogen, lower alkyl, lower hydroxyalkyl, lower alkoxyalkyl, lower haloalkyl, phenyl or a fused benzene ring, $R^6$ signifies hydrogen or lower alkyl, Ra signifies phenyl which is optionally substituted by one or two substitutens from the group consisting of lower alkyl, lower alkoxy, lower alkylthio, lower alkanoyl, lower alkoxycarbonyl, halogen, trifluoromethyl, hydroxy, nitro and cyano, Rb signifies lower alkyl, lower alkoxycarbonyl, halogen, trifluoromethyl, hydroxy, nitro and cyano, Rb signifies lower alkyl, lower alkoxy or phenyl which is optionally substituted by one or two substituents from the group consisting of lower alkyl, lower alkoxy, lower alkylthio, lower alkanoyl, lower alkoxycarbonyl, halogen, trifluoromethyl, hydroxy, nitro and cyano, Rc signifies hydrogen or lower alkyl, the dotted line signifies an additional double bond and the symbol $Y^-$ signifies a pharmaceutically acceptable anion, whereby $R^3$ has a significance different from methyl when $R^4$ signifies the group $-N=CH-Q-NR^1R^2$, $R^1$ and $R^2$ each signify methyl, $R^5$ and $R^6$ each signify hydrogen, Q signifies 1,4-phenylene and Y signifies chlorine, and the terms denoted by lower have a maximum of 7 carbon atoms, and pharmaceutically acceptable acid addition salts thereof.
2. Compounds in accordance with claim 1, wherein $R^3$ signifies lower alkyl, $R^4$ signifies the group $-N=CH-Q-NR^1R^2$, –N=CH–Ra or $-CH_2-CO-Rb$ and $R^5$ and $R^6$ each signify hydrogen.
3. Compounds in accordance with claim 1 or 2, wherein $R^4$ signifies the group $-N=CR^6-Q-NR^1R^2$.
4. Compounds in accordance with claim 3, wherein Q signifies 1,4-phenylene.
5. Compounds in accordance with any one of claims 1–4, wherein $R^1$ and $R^2$ each signify lower alkyl.
6. Compounds in accordance with any one of claims 1–5, wherein $R^3$ signifies lower alkyl with at least two carbon atoms.
7. Compounds in accordance with any one of claims 1, 2, 4, 5 and 6, wherein $R^4$ signifies the group –N=CRc–Ra or $-CH_2-CO-Rb$.
8. Compounds in accordance with any one of claims 1–7, wherein $R^5$ signifies hydrogen.
9. Compounds in accordance with any one of claims 1–8, wherein $R^6$ and Rc signify hydrogen.
10. 3-[p-(Chlorobenzylidene)amino]-1-[[p-(dimethylamino)benzylidene]amino]-2-ethylimidazolium salts.
11. 3-[p-(Dimethylamino)phenacyl]-1-[[p-(dimethylamino)benzylidene]amino]-2-ethylimidazolium salts.
12. 1,3-Bis[(p-(dimethylamino)benzylidene]amino]-2-ethylimidazolium salts.
13. 1[[p-(Dimethylamino)benzylidene]amino]-2-ethyl-3-[(p-nitrobenzylidene)amino]imidazolium salts.
14. 1,3-Bis[(p-(dimethylamino)benzylidene]amino]-2-propylimidazolium salts.
15. Compounds in accordance with any one of claims 1–14 for use as therapeutically active substances.
16. Compounds in accordance with any one of claims 1–14 for use as anthelmintically-active substances.

17. 1,3-Bis[[p-(dimethylamino)benzylidene]amino]-2-methylimidazolium chloride for use as a therapeutically active substance, especially for use as an anthelmintically-active substance.

18. A process for the manufacture of compounds in accordance with any one of claims 1–14, characterised by

a) reacting a compound of the general formula

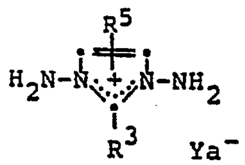

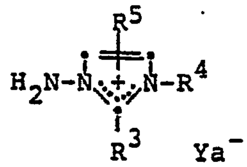

or

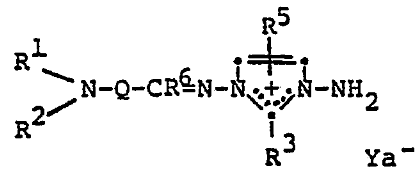

II III IIIa'

wherein $R^1$, $R^2$, $R^3$, $R^4$, $R^5$, $R^6$ and Q have the significance given in claim 1 and $Ya^-$ signifies an anion, with a carbonyl compound of the general formula

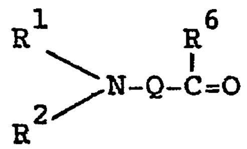

IVa

wherein Q, $R^1$, $R^2$ and $R^6$ have the significance given in claim 1, or reacting a compound of formula IIIa′ above with a carbonyl compound of the general formula

Ra–C(Rc)=O IVb

wherein Ra and Rc have the significance given in claim 1, or

b) reacting a compound of the general formula

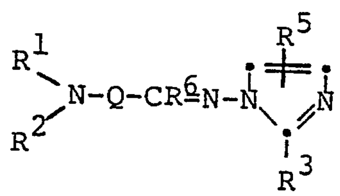

Va'

wherein $R^1$, $R^2$, $R^3$, $R^5$, $R^6$ and Q have the significance given in claim 1, with a compound of the general formula

$R^{41}$–X VI

wherein $R^{41}$ signifies the group $-CH_2-CO-Rb$, X signifies a leaving group and Rb has the significance given in claim 1.

c) optionally replacing the anion denoted by $Ya^-$ in a compound obtained by a pharmaceutically acceptable anion and

d) if desired, converting a compound of formula I obtained into a pharmaceutically acceptable salt.

19. Compounds of the general formula

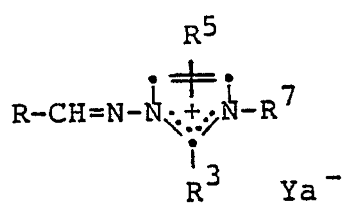

XV

wherein $R^7$ signifies the group $-CH_2-CO-Rb$, R signifies phenyl which is optionally substituted by lower

alkyl, lower alkoxy or halogen, Ya⁻ signifies an anion and $R^3$, $R^5$, Rb, the term "lower" and the dotted line have the significance given in claim 1.

20. Compounds of the general formula

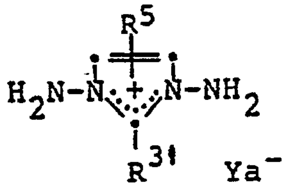

II'

wherein $R^{31}$ signifies lower alkyl with at least two carbon atoms, $Ya^-$ signifies an anion and $R^5$ and the dotted line have the significance given in claim 1.

21. Compounds of the general formula

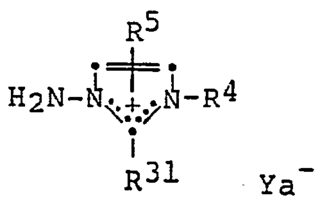

III'

wherein $R^{31}$ signifies lower alkyl with at least two carbon atoms, $Ya^-$ signifies an anion and $R^4$, $R^5$, the term "lower" and the dotted line have the significance given in claim 1.

22. Compounds of the general formula

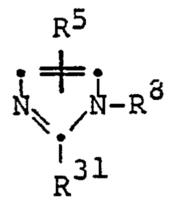

XVI'

wherein $R^8$ signifies the group $-N{=}CR^6-Q-NR^1R^2$ or $-N{=}CRc-Ra$, $R^{31}$ signifies lower alkyl with at least two carbon atoms and $R^1$, $R^2$, $R^5$, $R^6$, Q, Ra, Rc and the term "lower" have the significance given in claim 1.

23. The use of compounds in accordance with any one of claims 19–22 for the manufacture of compounds in accordance with any one of claims 1–14.

24. A medicament containing a compound in accordance with any one of claims 1–17 and a therapeutically inert carrier material.

25. An anthelmintically-active medicament containing a compound in accordance with any one of claims 1–17 and a therapeutically inert carrier material.

26. The use of compounds in accordance with any one of claims 1–17 for the manufacture of anthelmintically-active medicaments.

**Claims for the Contracting State: AT**

1. A process for the manufacture of compounds of the general formula

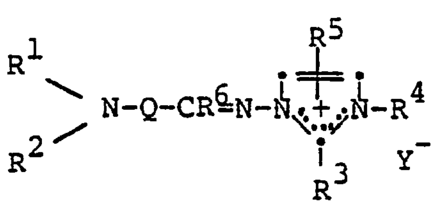

I

wherein the symbol Q signifies a 1,4-phenylene or 1,4-naphthylene group which can be substituted by one or two substitutents from the group consisting of lower alkyl and lower alkoxy, $R^1$ and $R^2$ each signify hydrogen or lower alkyl or together with the nitrogen atom signify 4-morpholinyl, 1-piperazinyl, 4-(lower alkyl)-1-piperazinyl, (4-lower alkoxycarbonyl)-1-piperazinyl or 1-pyrrolidinyl which is optionally substituted by one or two lower alkyl groups, $R^3$ signifies lower alkyl, $R^4$ signifies the group $-N{=}CR^6-Q-NR^1R^2$, $-N{=}CRc-Ra$ or $-CH_2-CO-Rb$, $R^5$ signifies hydrogen, lower alkyl, lower hydroxyalkyl, lower alkoxyalkyl, lower haloalkyl, phenyl or a fused benzene ring, $R^6$ signifies hydrogen or lower alkyl, Ra signifies phenyl which is optionally substituted by one or two substitutens from the group consisting of lower alkyl, lower alkoxy, lower alkylthio, lower alkanoyl, lower alkoxycarbonyl, halogen, trifluoromethyl, hydroxy, nitro and cyano, Rb signifies lower alkyl, lower alkoxy or phenyl which is optionally substituted by one or two substituents from the group consisting of lower alkyl, lower alkoxy, lower alkylthio, lower alkanoyl, lower alkoxycarbonyl, halogen, trifluoromethyl, hydroxy, nitro and cyano, Rc signifies hydrogen or lower alkyl, the dotted line signifies an additional double bond and the symbol $Y^-$ signifies a pharmaceutically acceptable anion, whereby $R^3$ has a significance different from methyl when $R^4$ signifies the group $-N{=}CH-Q-NR^1R^2$, $R^1$ and $R^2$ each signify methyl, $R^5$ and $R^6$ each signify hydrogen, Q signifies 1,4-phenylene and Y signifies chlorine, and the terms denoted by lower have a maximum of 7 carbon atoms, and of pharmaceutically acceptable acid addition salts thereof, characterized by

a) reacting a compound of the general formula

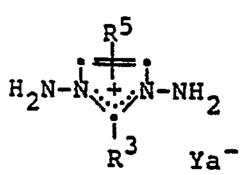

II

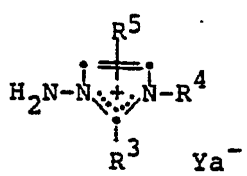

III

or

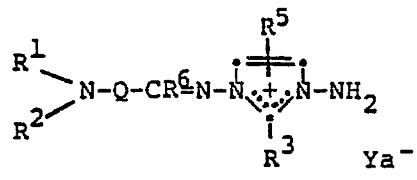

IIIa'

wherein $R^1$, $R^2$, $R^3$, $R^4$, $R^5$, $R^6$ and Q have the above significance and $Ya^-$ signifies an anion, with a carbonyl compound of the general formula

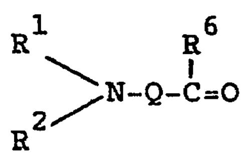

IVa

wherein Q, $R^1$, $R^2$ and $R^6$ have the above significance or reacting a compound of formula IIIa' above with a carbonyl compound of the general formula

$$Ra-C(Rc){=}O \qquad IVb$$

wherein Ra and Rc have the above significance, or

b) reacting a compound of the general formula

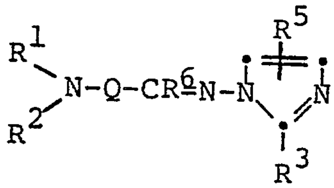

Va'

wherein $R^1$, $R^2$, $R^3$, $R^5$, $R^6$ and Q have the above significance, with a compound of the general formula

$$R^{41}-X \qquad VI$$

wherein $R^{41}$ signifies the group $-CH_2-CO-Rb$, X signifies a leaving group and Rb has the above significance,

c) optionally replacing the anion denoted by $Ya^-$ in a compound obtained by a pharmaceutically acceptable anion and

d) if desired, converting a compound of formula I obtained into a pharmaceutically acceptable salt.

2. A process in accordance with claim 1, wherein $R^3$ signifies lower alkyl, $R^4$ signifies the group $-N{=}CH-Q-NR^1R^2$, $-N{=}CH-Ra$ or $-CH_2-CO-Rb$ and $R^5$ and $R^6$ each signify hydrogen.

3. A process in accordance with claim 1 or 2, wherein $R^4$ signifies the group $-N{=}CR^6-Q-NR^1R^2$.

4. A process in accordance with claim 3, wherein Q signifies 1,4-phenylene.

5. A process in accordance with any one of claims 1–4, wherein $R^1$ and $R^2$ each signify lower alkyl.

6. A process in accordance with any one of claims 1–5, wherein $R^3$ signifies lower alkyl with at least two carbon atoms.

7. A process in accordance with any one of claims 1, 2, 4, 5 and 6, wherein $R^4$ signifies the group $-N{=}CRc{-}Ra$ or $-CH_2{-}CO{-}Rb$.

8. A process in accordance with any one of claims 1–7, wherein $R^5$ signifies hydrogen.

9. A process in accordance with any one of claims 1–8, wherein $R^6$ and Rc signify hydrogen.

10. A process in accordance with claim 1, characterized in that 3-[p-(chlorobenzylidene)amino]-1-[[p-(dimethylamino)benzylidene]amino]-2-ethylimidazolium salts are manufactured.

11. A process in accordance with claim 1, characterized in that 3-[p-(dimethylamino)phenacyl]-1-[[p-(dimethylamino)benzylidene]amino]-2-ethylimidazolium salts are manufactured.

12. A process in accordance with claim 1, characterized in that 1,3-bis[[p-(dimethylamino)benzylidene]-amino]-2-ethylimidazolium salts are manufactured.

13. A process in accordance with claim 1, characterized in that 1[[p-(dimethylamino)benzylidene]amino]-2-ethyl-3-[(p-nitrobenzylidene)amino]imidazolium salts are manufactured.

14. A process in accordance with claim 1, characterized in that 1,3-bis[(p-(dimethylamino)benzylidene]-amino]-2-propylimidazolium salts are manufactured.

15. The use of compounds in accordance with any one of claims 1–14 for the manufacture of anthelmintically-active medicaments.

16. The use of 1,3-bis[[p-(dimethylamino)benzylidene]amino]-2-methylimidazolium chloride for the manufacture of anthelmintically-active medicaments.

**Revendications pour les Etats Contractants: BE, CH, DE, FR, GB, IT, LI, NL**

1. Composés de formule générale

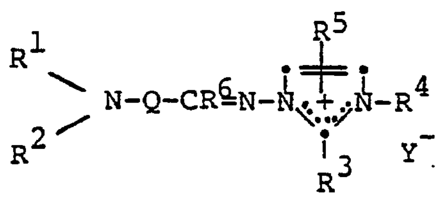

I

où le symbole Q représente un groupe 1,4-phénylène ou un groupe 1,4-naphtylène, qui peuvent être aussi substitués par un ou deux substituants choisis dans le groupe constitué par un alkyle inférieur et un alcoxy inférieur, $R^1$ et $R^2$ représentent chacun l'hydrogène, ou un alkyle inférieur, ou ensemble avec l'atome d'azote un 4-morpholinyle, un 1-pipérazinyle, un 4-(alkyl inférieur)-1-pipérazinyle, un (4-alcoxycarbonyl inférieur)-1-pipérazinyle ou un 1-pyrrolidinyle éventuellement substitués par un ou deux groupes alkyles inférieurs, $R^3$ représente un alkyle inférieur, $R^4$ représente le groupe $-N{=}CR^6{-}Q{-}NR^1R^2$, $-N{=}CRc{-}Ra$ ou $-CH_2{-}CO{-}Rb$, $R^5$ représente l'hydrogene, un alkyle inférieur, un hydroxyalkyle inférieur, un alcoxyalkyle inférieur, un halogénoalkyle inférieur, un phényle ou un cycle benzol fusionné, $R^6$ représente l'hydrogène ou un alkyle inférieur, Ra représente un phényle éventuellement substitué par un ou deux substituants choisis dans le groupe constitué par un alkyle inférieur, un alcoxy inférieur, un alkylthio inférieur, un alcanoyle inférieur, un alcoxycarbonyle inférieur, un halogène, un trifluorométhyle, un hydroxy, un nitro et un cyano, Rb représente un alkyle inférieur, un alcoxy inférieur ou un phényle éventuellement substitué par un ou deux substituants choisis dans le groupe constitué par un alkyle inférieur, un alcoxy inférieur, un alkylthio inférieur, un alcanoyle inférieur, un alcoxycarbonyle inférieur, un halogène, un trifluorométhyle, un hydroxy, un nitro et un cyano, Rc représente l'hydrogène ou un alkyle inférieur, la ligne en pointillés représente une double liaison supplémentaire, et le symbole $Y^-$ représente un anion acceptable pharmaceutiquement, $R^3$ ayant une signification différente d'un méthyle, lorsque $R^4$ représente le groupe $-N{=}CH{-}Q{-}NR^1R^2$, $R^1$ et $R^2$ représentent chacun un méthyle, $R^5$ et $R^6$ représentent chacun l'hydrogène, Q représente le 1,4-phénylène et Y représente le chlore, et où les termes avec la dénomination "inférieur" comportent au plus 7 atomes de carbone, ainsi que leur sels d'addition d'acides acceptables pharmaceutiquement.

2. Composés selon la revendication 1, où $R^3$ représente un alkyle inférieur, $R^4$ représente le groupe $-N{=}CH{-}Q{-}NR^1R^2$, $-N{=}CH{-}Ra$, ou $-CH_2{-}CO{-}Rb$ et $R^5$ et $R^6$ représentent chacun l'hydrogène.

3. Composés selon la revendication 1 ou 2 où $R^4$ représente le groupe $-N{=}CR^6{-}Q{-}NR^1R^2$.

4. Composé selon la revendication 3 où Q représente le 1,4-phénylène.

5. Composé selon l'une des revendications 1 à 4, où $R^1$ et $R^2$ représentent chacun un alkyle inférieur.

6. Composés selon l'une des revendications 1 à 5, où $R^3$ représente un alkyle inférieur avec au moins deux atomes de carbone.

7. Composés selon l'une des revendications 1, 2, 4, 5 et 6, où $R^4$ représente le groupe –N=CRc–Ra ou $–CH_2–CO–Rb$.

8. Composés selon l'une des revendications 1 à 7, où $R^5$ représente l'hydrogène.

9. Composés selon l'une des revendications 1 à 8, où $R^6$ et Rc représentent l'hydrogène.

10. Sels de 3-[p-(chlorobenzylidène)amino]-1-[[p-(diméthylamino)benzylidène]amino]-2-éthylimidazolium.

11. Sels de 3-[p-(diméthylamino)phénacyl]-1-[[p-(diméthylamino)-benzylidène]amino]-2-éthylimidazolium.

12. Sels de 1,3-[p-(diméthylamino)benzylidène]amino]-2-éthylimidazolium.

13. Sels de 1-[[p-(diméthylamino)benzylidène]amino]-2-éthyl-3-[(p-nitrobenzylidène)amino]imidazolium.

14. Sels de 1,3-bis[[p-(diméthylamino)benzylidène]amino]-2-propylimidazolium.

15. Composé selon l'une des revendications 1 à 14 pour utilisation comme matière active thérapeutique.

16. Composé selon l'une des revendications 1 à 14 pour utilisation comme matière active anthelmintique.

17. Chlorure de 1,3-bis-[[p-(diméthylamino)benzylidène]amino]-2-méthylimidazolium pour utilisation comme matière active thérapeutique, en particulier pour utilisation comme matière active anthelmintique.

18. Procédé pour produire des composés selon l'une des revendications 1 à 14, caractérisé en ce que

a) on fait réagir un composé de formule générale

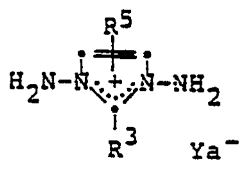

II

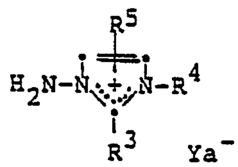

III

ou

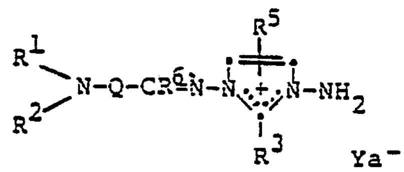

IIIa'

où $R^1$, $R^2$, $R^3$, $R^4$, $R^5$, $R^6$ et Q ont la signification donnée à la revendication 1, et Ya– est un anion, avec un composé carbonyle de formule générale

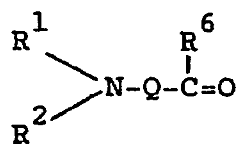

IVa

où Q, $R^1$, $R^2$ et $R^6$ ont la signification donnée à la revendication 1, ou on fait réagir un composé ayant la formule précédente IIIa' avec un composé carbonyle de formule générale

Ra–C(Rc)=O IVb

où Ra et Rc ont la signification donnée à la revendication 1, ou

b) on fait réagir un composé de formule générale

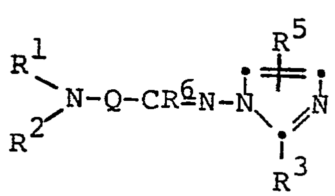

Va'

où $R^1$, $R^2$, $R^3$, $R^5$, $R^6$ et Q ont la signification donnée à la revendication 1, avec un composé de formule générale

$R^{41}$–X VI

où $R^{41}$ représente le groupe $–CH_2–CO–Rb$ et X représente un groupe de départ, et Rb a la signification donnée à la revendication 1,

c) on échange éventuellement l'anion désigné par $Ya^-$, dans un composé obtenu, contre un anion acceptable pharmaceutiquement, et

d) si on le désire, on transforme un composé obtenu de formule I en un sel pharmaceutiquement acceptable.

19. Composés de formule générale

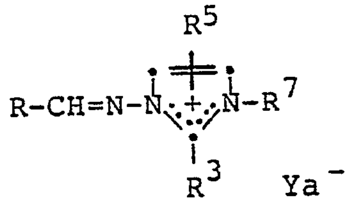

XV

où $R^7$ représente le groupe $-CH_2-CO-Rb$, R représente un phényle éventuellement substitué par un alkyle inférieur, un alcoxy inférieur ou un halogène, et $Ya^-$ représente un anion, et $R^3$, $R^5$, Rb, l'expression "inférieur", et la ligne en pointillés ont la signification donnée à la revendication 1.

20. Composés de formule générale

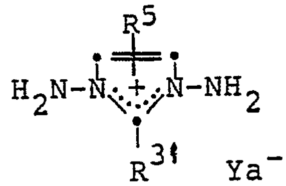

II'

où $R^{31}$ représente un alkyle inférieur avec au moins deux atomes de carbone et $Ya^-$ représente un anion, et $R^5$ et la ligne en pointillés ont la signification donnée à la revendication 1.

21. Composés de formule générale

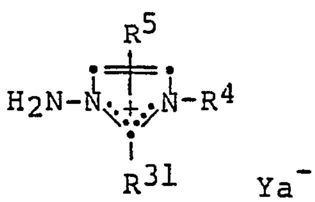

III'

où $R^{31}$ représente un alkyle inférieur avec au moins deux atomes de carbone et $Ya^-$ représente un anion, et $R^5$, l'expression "inférieur" et la ligne en pointillés ont la signification donnée à la revendication 1.

22. Composés de formule générale

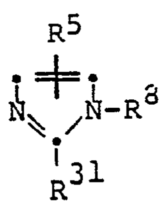

XVI'

où $R^8$ représente le groupe $-N=CR^6-Q-NR^1R^2$ ou $-N=CRc-Ra$ et $R^{31}$ représente un alkyle inférieur avec au moins deux atomes de carbone, et $R^1$, $R^2$, $R^5$, $R^6$, Q, Ra, Rc et l'expression "inférieur" ont la signification donnée à la revendication 1.

23. Utilisation de composés selon l'une des revendications 19 à 22 pour produire des composés selon l'une des revendications 1 à 14.

24. Médicaments contenants un composé selon l'une des revendications 1 à 17 et un support thérapeutiquement inerte.

25. Agents actifs anthelmintique comportant un composé selon l'une des revendications 1 à 17 et un support thérapeutiquement inerte.

26. Utilisation de composés selon l'une des revendications 1 à 17 pour produire des agents actifs anthelmintiques.

**Revendications pour l'Etat Contractant: AT**

1. Procédé pour préparer des composés de formule générale

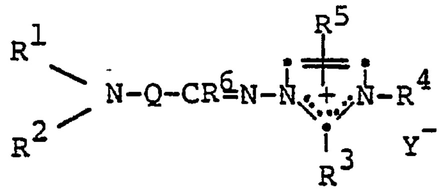

I

où le symbole Q représente un groupe 1,4-phénylène ou un groupe 1,4-naphtylène, qui peuvent être aussi substitués par un ou deux substituants choisis dans le groupe constitué par un alkyle inférieur et un alcoxy inférieur, $R^1$ et $R^2$ représentent chacun l'hydrogène, ou un alkyle inférieur, ou ensemble avec l'atome d'azote un 4-morpholinyle, un 1-pipérazinyle, un 4-(alkyl inférieur)-1-pipérazinyle, un (4-alcoxycarbonyl inférieur)-1-pipérazinyle ou un 1-pyrrolidinyle éventuellement substitués par un ou deux groupes alkyles inférieurs, $R^3$ représente un alkyle inférieur, $R^4$ représente le groupe $-N=CR^6-Q-NR^1R^2$, $-N=CRc-Ra$ ou $-CH^2-CO-Rb$, $R^5$ représente l'hydrogene, un alkyle inférieur, un hydroxyalkyle inférieur, un alcoxyalkyle inférieur, un halogénoalkyle inférieur, un phényle ou un cycle benzol fusionné, $R^6$ représente l'hydrogène ou un alkyle inférieur, Ra représente un phényle éventuellement substitué par un ou deux substituants choisis dans le groupe constitué par un alkyle inférieur, un alcoxy inférieur, un alkylthio inférieur, un alcanoyle inférieur, un alcoxycarbonyle inférieur, un halogène, un trifluorométhyle, un hydroxy, un nitro et un cyano, Rb représente un alkyle inférieur, un alcoxy inférieur ou un phényle éventuellement substitué par un ou deux substituants choisis dans le groupe constitué par un alkyle inférieur, un alcoxy inférieur, un alkylthio inférieur, un alcanoyle inférieur, un alcoxycarbonyle inférieur, un halogène, un trifluorométhyle, un hydroxy, un nitro et un cyano, Rc représente l'hydrogène ou un alkyle inférieur, la ligne en pointillés représente une double liaison supplémentaire, et le symbole $Y^-$ représente un anion acceptable pharmaceutiquement, $R^3$ ayant une signification différente d'un méthyle, lorsque $R^4$ représente le groupe $-N=CH-Q-NR^1R^2$, $R^1$ et $R^2$ représentent chacun un méthyle, $R^5$ et $R^6$ représentent chacun l'hydrogène, Q représente le 1,4-phénylène et Y représente le chlore, et où les termes avec la dénomination "inférieur" comportent au plus 7 atomes de carbone, et de leurs sels d'addition d'acides acceptables pharmaceutiquement, caractérisé en ce que

a) on fait réagir un composé de formule générale

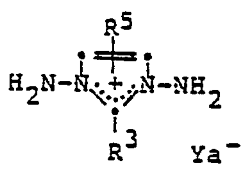

II

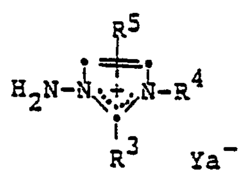

III

ou

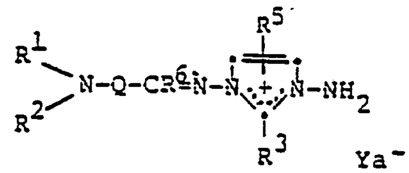

IIIa'

où $R^1$, $R^2$, $R^3$, $R^4$, $R^5$, $R^6$ et Q ont la signification donné précédemment, et $Ya^-$ représente un anion, avec un composé carbonyle de formule générale

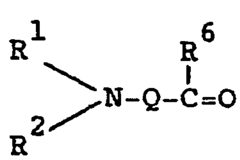

IVa

où Q, $R^1$, $R^2$ et $R^6$ ont la signification donnée précédemment, ou on fait réagir un composé ayant la formule précédente IIIa' avec un composé carbonyle de formule générale

Ra–C(Rc)=O   IVb

où Ra et Rc ont la signification donnée précédemment, ou

b) on fait réagir un composé de formule générale

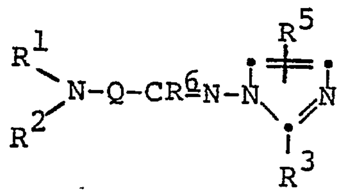

Va'

où $R^1$, $R^2$, $R^3$, $R^5$, $R^6$ et Q ont la signification donnée précédemment avec un composé de formule générale

$$R^{41}\text{–}X \qquad VI$$

où $R^{41}$ représente le groupe $-CH_2-CO-Rb$ et X représente un groupe de départ, et Rb a la signification donnée précédemment,

c) on échange éventuellement l'anion désigné par $Ya^-$, dans un composé obtenu par un anion pharmaceutiquement acceptable, et

d) si on le désire, on transforme un composé obtenu de formule I en un sel pharmaceutiquement acceptable.

2. Procédé selon la revendication 1, où $R^3$ représente un alkyle inférieur, $R^4$ représente le groupe $-N=CH-Q-NR^1R^2$, $-N=CH-Ra$ ou $-CH^2-CO-Rb$, et $R^5$ et $R^6$ représentent chacun l'hydrogène.

3. Procédé selon la revendication 1 ou 2, où $R^4$ représente le groupe $-N=CR^6-Q-NR^1R^2$.

4. Procédé selon la revendication 3, où Q représente le 1,4-phénylène.

5. Procédé selon l'une des revendications 1 à 4, où $R^1$ et $R^2$ représentent chacun un alkyle inférieur.

6. Procédé selon l'une des revendications 1 à 5, où $R^3$ représente un alkyle inférieur avec au moins deux atomes de carbone.

7. Procédé selon l'une des revendications 1, 2, 4, 5 et 6 où $R^4$ représente le groupe $-N=CRc-Ra$ ou $-CH_2-CO-Rb$.

8. Procédé selon l'une des revendications 1 à 7, où $R^5$ représente l'hydrogène.

9. Procédé selon l'une des revendications 1 à 8, où $R^6$ et Rc représentent l'hydrogène.

10. Procédé selon la revendication 1, caractérisé en ce que l'on prépare des sels de 3-[p-(chlorobenzylidène)amino]-1-[[p-(diméthylamino)benzylidène]amino]-2-éthylimidazolium.

11. Procédé selon la revendication 1, caractérisé en ce que l'on prépare des sels de 3-[p-(diméthylamino)phénacyl]-1-[[p-(diméthylamino)benzylidène]amino]-2-éthylimidazolium.

12. Procédé selon la revendication 1, caractérisé en ce que l'on prépare des sels de 1,3-bis[p-(diméthylamino)benzylidène]amino]-2-éthylimidazolium.

13. Procédé selon la revendication 1, caractérisé en ce que l'on prépare des sels de 1-[[p-(diméthylamino)benzylidène]amino]-2-éthyl-3-[(p-nitrobenzylidène)amino]imidazolium.

14. Procédé selon la revendication 1, caractérisé en ce que l'on prépare des sels de 1,3-bis[[p-(diméthylamino)benzylidène]amino]-2-propylimidazolium.

15. Utilisation de composés selon l'une des revendications 1 à 14 pour produire des agents actifs anthelmintiques.

16. Utilisation de chlorure de 1,3-bis[[p-(diméthylamino)benzylidène]amino]-2-méthylimidazolium pour produire des agents actifs anthelmintiques.